# EUROPEAN PATENT APPLICATION

(11) **EP 4 464 412 A2**
(43) Date of publication of application: **20.11.2024**
(21) Application number: 24194384.4
(22) Date of filing: 14.09.2018
(51) Int. Cl.: B01L 3/00

(54) **DETECTION OF RECOMBINASE POLYMERASE AMPLIFICATION USING DUAL-HAPTEN PROBE**

(30) Priority: 14.09.2017 US 201762558705 P
(62) Divisional of application: 18856152.6
(71) Applicant: Abbott Diagnostics Scarborough, Inc., Scarborough, Maine 04074-8303 (US)
(72) Inventor: POWELL, Michael L., San Diego, 92121 (US); BOWLER, Frank Ray, San Diego, 92121 (US); GREENWOOD, Catherine Jean, San Diego, 92121 (US); PIEPENBURG, Olaf, San Diego, 92121 (US); ARMES, Niall A., San Diego, 92121 (US)
(74) Representative: Mathys & Squire

(57) **Abstract**

This disclosure relates to methods and compositions for detecting a target nucleic acid sequence using a dual-hapten probe. More specifically, the present disclosure relates to using recombinase polymerase amplification (RPA) and a dual-hapten probe to detect a target nucleic acid sequence. In some cases, the detection is on lateral flow strips.

## Description

### CROSS-REFERENCE TO RELATED APPLICATION

This application claims the benefit of U.S. Patent Application Serial No. 62/558,705 entitled "DETECTION OF RECOMBINASE POLYMERASE AMPLIFICATION USING DUAL-HAPTEN PROBE" filed September 14, 2017, which is hereby incorporated by reference in its entirety.

### TECHNICAL FIELD

This disclosure relates to methods and compositions for detecting a target nucleic acid sequence using a dual-hapten probe. More specifically, the present disclosure relates to methods and compositions of using recombinase polymerase amplification (RPA) and a dual-hapten probe to detect a target nucleic acid sequence. In some cases, the detection is on lateral flow strips.

### BACKGROUND

Certain isothermal amplification methods are able to amplify target nucleic acid from trace levels to very high and detectable levels within a matter of minutes. Such isothermal methods, e.g., Recombinase Polymerase Amplification (RPA), can allow users to detect a particular sequence in trace amounts, facilitating point-of-care testing and increasing the accessibility and speed of diagnostics.

RPA has been shown to be suitable for use in non-laboratory settings, with reported sensitivities comparable to PCR-based diagnostics and real-time detection of target DNA. However, these assays remain more suitable for use in laboratory settings or with a 'lab-in-a-suitcase' type setup. A number of groups have therefore developed lateral flow assays, for situations in which only qualitative data is required. Lateral flow tests are relatively simple to perform for untrained personnel/home use and are one of the preferred formats for use in resource-limited settings as they do not require expensive equipment to perform. The lateral flow technology, however, still requires a number of manipulations, including dilution steps prior to lateral flow analysis. There remains the need for a simplified approach to the use of RPA amplification and lateral flow detection, by reducing the number of manipulations required. This improvement would present benefits in the manufacture of consumables for RPA lateral flow assays, permitting simplification of the test device, and thus reduced consumable costs, making such assays more suitable for use outside of the laboratory.

### SUMMARY

This disclosure is based, at least in part, on the discovery that RPA of a target nucleic acid sequence can be accurately and efficiently detected on a lateral flow strip with no dilution step, using a dual-hapten probe. In view of this discovery, provided herein are RPA compositions and methods for detecting the presence or absence of a target nucleic acid using a dual-hapten probe. These target nucleic acid sequences can be diagnostic of disease or disorder.

In one aspect, this disclosure features a recombinase polymerase amplification composition comprising, consisting of, or consisting essentially of a crowding agent; an oligonucleotide probe with a dual-hapten leaving group; and a nuclease enzyme. In another aspect, this disclosure features a recombinase polymerase amplification composition for use in lateral flow analysis of a target nucleic acid present in a sample which requires no dilution of the amplification mixture prior to separation of amplification products on a lateral flow test strip, comprising, consisting of, or consisting essentially of a crowding agent; an oligonucleotide probe with a dual-hapten leaving group; and a nuclease enzyme.

In some embodiments of all aspects, the crowding agent of the compositions and methods described herein comprises, consists of, or consists essentially of polyethylene glycol (PEG), polyvinyl alcohol (PVA), polyvinylpyrrolidone (PVP), Ficoll, or dextran. In some embodiments of all aspects, the crowding agent has a molecular weight of at least 1 kilodalton, at least 2 kilodaltons, at least 3 kilodaltons, at least 4 kilodaltons, at least 5 kilodaltons, at least 6 kilodaltons, at least 8 kilodaltons, or at least 10 kilodaltons. In some embodiments of all aspects, the crowding agent is present in the composition at a concentration of at least 15% v/v, a concentration of at least 12% v/v, a concentration of at least 10% v/v, a concentration of at least 8% v/v, at least 6% v/v, a concentration of at least 5% v/v, a concentration of at least 4% v/v, or a concentration of at least 3% v/v. In some embodiments of all aspects, the crowding agent has a viscosity profile at 20 degrees Celsius of less than or equal to 5mPa/s, less than or equal to 4mPa/s, less than or equal to 3mPa/s, less than or equal to 2mPa/s, or less than or equal to 1mPa/s. In some embodiments of all aspects, the crowding agent is PEG having a viscosity at 20 degrees Celsius is less than or equal to 3 mPa/s. In some embodiments of all aspects, the crowding agent is PEG having a molecular weight of 3 kilodaltons, and wherein the PEG is at concentration of 6.5% v/v.

In some embodiments of all aspects, the oligonucleotide probe of the compositions and methods described herein comprises a dR-O-[C]n nucleotide that lacks a base linking the leaving group to the oligonucleotide. In some embodiments of all aspects, the oligonucleotide probe with a dual-hapten, when cleaved by formamidopyrimidine DNA glycosylase when the oligonucleotide probe is hybridised to a complementary nucleotide sequence, releases the dual-hapten leaving group. In some embodiments of all aspects, the dual-hapten leaving group comprises, consists of, or consists essentially of two immunogenic groups having different epitopes. In some embodiments of all aspects, the immunogenic groups comprise, consist of, or consist essentially of a fluorescent group, an enzyme or fragment thereof, a peptide or fragment thereof, biotin. In some embodiments of all aspects, the immunogenic groups are selected from the group comprising biotin, fluorescein, digoxigenin or dinitrophenyl.

In some embodiments of all aspects, the nuclease of the compositions and methods described herein is formamidopyrimidine DNA glycosylase.

In another aspect, this disclosure features compositions comprising, consisting of, or consisting essentially of: 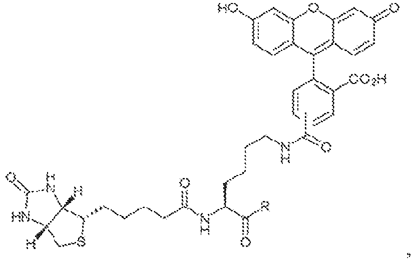 wherein R is OH or -NH(CH2)₆OH.

In another aspect, this disclosure features compositions comprising, consisting of, or consisting essentially of: 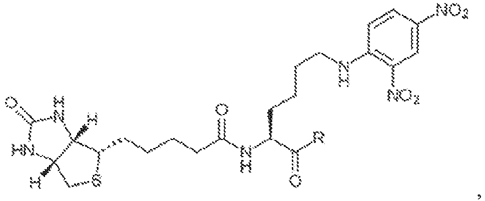 wherein R is OH or - NH(CH2)₆OH.

In another aspect, this disclosure features compositions comprising, consisting of, or consisting essentially of: 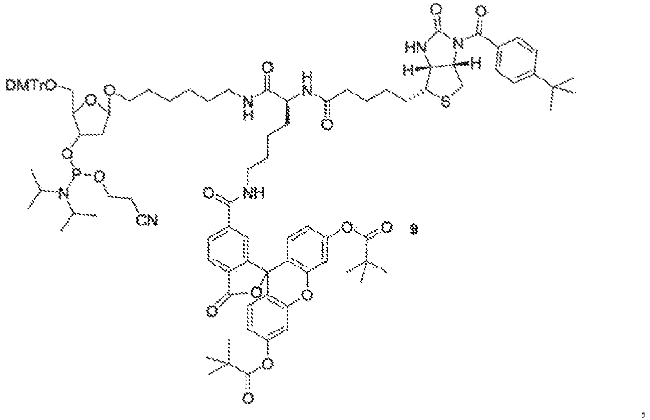 wherein where DMTr is dimethoxytrityl.

In another aspect, this disclosure features compositions comprising, consisting of, or consisting essentially of: 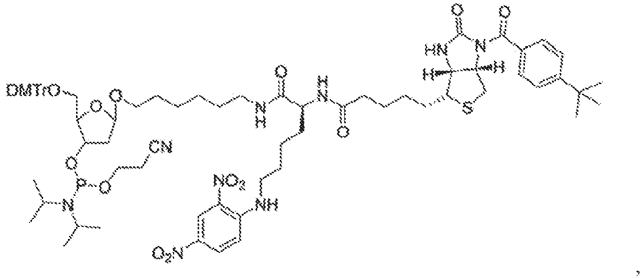 wherein DMTr is dimethoxytrityl.

In another aspect, this disclosure features compositions comprising, consisting of, or consisting essentially of: 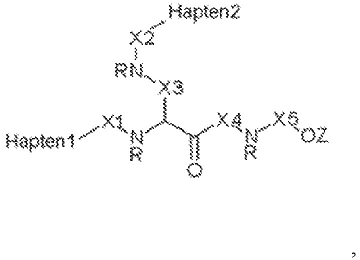 wherein: Hapten1 and Hapten2 are immunogenic groups as described herein; Z is selected from: (i) a C1' of an abasic ribose or deoxyribose ring within the context of an RNA or DNA oligonucleotide respectively; with a beta-configuration at the anomeric carbon atom; (ii) a phosphoramidite compound configured to link into a DNA or RNA oligonucleotide; and wherein when Z is a DNA or RNA phosphoramidite, the reactive groups of Hapten1 and Hapten2 may optionally be protected with pivaloyl; tert-butylbenzoyl; acyl; benzoyl; or isobutyryl; R represents hydrogen, or linear or branched C1 to C6 alkyl; X1, X2 and X4 are linking groups which may independently be absent, or be linear or branched C1 to C12 alkyl, which may be optionally interrupted by one or more -0-, -C(=O)- or -NR- groups; X3 is linear or branched C1 to C6 alkyl; and X5 is linear or branched C1 to C12 alkyl, which is optionally interrupted by one or more -0-, -C(=O)- or -NR- groups.

In some embodiments, the oligonucleotide probe is cleavable by an exonuclease, and the oligonucleotide releases the dual hapten leaving group when cleaved. In some embodiments, the exonuclease is exonuclease III. In some embodiments, the oligonucleotide probe has the structure 5'X(n)ₐL(n)_{b}H(n)_{c}B3' wherein n are nucleotides a, b, and c are integers, X is a 5' hexyl or a hapten, H is a THF residue, B is a C3 spacer, and L is a branched modifier comprising a plurality of haptens. In some embodiments, the haptens are, for example DNP and Biotin, although other haptens may be utilized (e.g., FAM). In some embodiments, the oligonucleotide probe comprises a phosphorothioate link between the haptens. In some embodiments, a and c are at least 15 nucleotides. In some embodiments, b is zero. In some embodiments, a is approximately 15 and c is approximately 30. In some embodiments, the oligonucleotide probe is complementary to target nucleic acid. In some embodiments, L replaces a cytosine nucleotide.

In another aspect, this disclosure features devices comprising, consisting of, or consisting essentially of a lateral flow strip, comprising, consisting of, or consisting essentially of a sample application zone; a reagent zone downstream of the sample application zone and in fluid communication therewith, the reagent zone comprising dried RPA reagent composition for amplifying a target nucleic acid, a binding agent specific for an amplified target nucleic acid product and a detection molecule; at least one test zone downstream of the reagent zone and in fluid communication therewith, the test zone comprising an immobilized capture molecule specific for the amplified target nucleic acid product; and a control zone downstream of the test zone. In some embodiments, the devices provide for continuous (e.g., concurrent) RPA and detection. In some embodiments, devices comprise a heavyweight absorbent pad in the test zone.

In some embodiments of all aspects, the dried RPA reagent composition comprises a crowding agent, a recombinase, a polymerase, a nuclease, a dual-hapten probe and a detection molecule. In some embodiments of all aspects, the dual-hapten probe comprises a conjugate of biotin and carboxyfluorescein (FAM) or biotin and dinitrophenyl (DNP). In some embodiments of all aspects, the immobilised capture molecule specific for the amplified target nucleic acid is selected from the group consisting of an anti-FAM capture molecule or an anti-DNP capture molecule. In some embodiments of all aspects, the anti-FAM or the anti-DNP are independently selected from the group consisting of a polyclonal antibody, a monoclonal antibody, and a functional binding fragment thereof including a FAB, a ScFv, a Fv or a DAB. In some embodiments of all aspects, the detection molecule is selected from the group consisting of a gold sol, a silver sol, a latex sol, a cellulose nanobead, or a carbon nanostring, and an anti-biotin capture molecule.

In some embodiments of all aspects, the control zone comprises a binding zone that indicates correct operation of the lateral flow strip. In some embodiments of all aspects, the control zone comprises an anti-mouse antibody capture line.

In another aspect, this disclosure features methods of detecting amplification products, comprising, consisting of, or consisting essentially of contacting a sample suspected of containing a target nucleic acid of interest with RPA reagents for amplifying the target nucleic acid, an oligonucleotide probe comprising a nucleic acid sequence complementary to the target nucleic acid and a covalently linked dual-hapten leaving group, and a nuclease; amplifying the target nucleic acid to produce a target nucleic acid product; and detecting the target nucleic acid product by detecting free dual-hapten moieties cleaved from the oligonucleotide probe hybridized to the target nucleic acid.

In some embodiments of all aspects, the RPA reagents are located on a sample application zone of a lateral flow strip. In some embodiments of all aspects, nucleic acid amplification is performed on the lateral flow strip upon contact of the sample with the RPA reagents to form a nucleic acid amplification mixture. In some embodiments of all aspects, nucleic acid amplification is performed on the lateral flow strip without dilution or addition of other liquid to the RPA mixture. In some embodiments, the RPA reaction and the detection are concurrent

In some embodiments of all aspects, the dual-hapten moiety cleaved from the oligonucleotide probe is selectively captured at a test zone on the lateral flow located downstream of the sample application zone. In some embodiments of all aspects, the oligonucleotide probe comprising a covalently linked dual-hapten is not selectively captured at a test zone on the lateral flow strip. In some embodiments of all aspects, the lateral flow strip comprises, consists of, or consists essentially of a test zone and a control zone, wherein the test zone comprises, consists of, or consists essentially of a binding pair member for capture of dual-hapten cleaved from oligonucleotide and the control zone comprises a binding pair member for an internal control. In some embodiments of all aspects, the control zone comprises, consists of, or consists essentially of an anti-mouse antibody or a fragment thereof. In some embodiments of all aspects, the test zone comprises, consists of, or consists essentially of an anti-DNP capture molecule or an anti-FAM capture molecule. In some embodiments of all aspects, the anti-FAM capture molecule is selected from the group consisting of a monoclonal antibody, a polyclonal antibody, and a functional binding fragment thereof. In some embodiments of all aspects, the anti-DNP capture molecule is selected from the group consisting of a monoclonal antibody, a polyclonal antibody, or a functional binding fragment thereof.

In some embodiments of all aspects, detecting amplification products comprises, consists of, or consists essentially of capturing dual-hapten leaving group in a test zone and labelling captured dual-hapten leaving group with a detection molecule. In some embodiments of all aspects, the detection molecule is selected from the group consisting of a gold sol, a silver sol, a latex sol, a cellulose nanobead, and a carbon nanostring.

In another aspect, this disclosure features a method comprising, consisting of, or consisting essentially of applying a sample suspected of containing the target nucleic acid to a lateral flow strip; contacting the sample with a RPA reagent mixture for amplifying the target nucleic acid dried onto a reagent zone of the lateral flow strip; and detecting amplification products, if present, on a test zone of the lateral flow strip.

In some embodiments of all aspects, the sample suspected of containing a target nucleic acid is applied to an application zone of a lateral flow strip. In some embodiments of all aspects, the RPA reagent mixture comprises, consists of, or consists essentially of a crowding agent, a recombinase, a polymerase, a nuclease, and a dual-hapten oligonucleotide probe.

In some embodiments of all aspects, the crowding agent is selected from the group consisting of polyethylene glycol (PEG), polyvinyl alcohol (PVA), polyvinylpyrrolidone (PVP), Ficoll, and dextran. In some embodiments of all aspects, the crowding agent has a molecular weight of at least 1 kilodalton, at least 2 kilodaltons, at least 3 kilodaltons, at least 4 kilodaltons, at least 5 kilodaltons, at least 6 kilodaltons, at least 8 kilodaltons, or at least 10 kilodaltons. In some embodiments of all aspects, the crowding agent is present in the mixture at a concentration of at least 15% v/v, at least 12% v/v final concentration, at least 10% v/v, at least 8% v/v, at least 6% v/v, at least 5% v/v, at least 4% v/v, or at least 3% v/v final concentration. In some embodiments of all aspects, the crowding agent has a viscosity profile at 20 degrees Celsius of less than or equal to 5mPa/s, less than or equal to 4mPa/s, less than or equal to 3mPa/s, less than or equal to 2mPa/s, or less than or equal to 1mPa/s. In some embodiments of all aspects, the crowding agent is PEG and has a viscosity at 20 degrees Celsius of less than or equal to 3 mPa/s. In some embodiments of all aspects, the crowding agent is PEG comprisng molecular weight of 3 kilodaltons and wherein the PEG is at a final concentration of 6.5% v/v.

In some embodiments of all aspects, detecting amplification products, if present, comprises, consists of, or consists essentially of detecting a dual-hapten moiety cleaved from the oligonucleotide probe hybridized to the amplification products. In some embodiments of all aspects, dilution of the RPA mixture is not required prior to detection of amplification products.

The term "one or more" or "at least one" as used in the present disclosure stands for 1, 2, 3, 4, 5, 6, 7, 8, 9, 10 compounds or even more.

A "sample" as used herein refers to a biological material that is isolated from its environment (e.g., blood or tissue from an animal, cells, or conditioned media from tissue culture) and is suspected of containing, or known to contain an analyte or other desired material. A sample can also be a partially purified fraction of a tissue or bodily fluid, e.g., from a subject having a specific disease or condition. A reference sample can be a "normal" sample, from a donor not having the disease or condition. A reference sample can also be from an untreated donor or cell culture not treated with an active agent (e.g., no treatment or administration of vehicle only) or not subjected to conditions to induce a disease state. A reference sample can also be taken at a "zero time point" prior to contacting the cell with the agent to be tested.

The section headings used herein are for organizational purposes only and are not to be construed as limiting the described subject matter in any way. When definitions of terms in incorporated references appear to differ from the definitions provided in the present teachings, the definition provided in the present teachings shall control. It will be appreciated that there is an implied "about" prior to metrics such as temperatures, concentrations, and times discussed in the present teachings, such that slight and insubstantial deviations are within the scope of the present teachings herein. In this application, the use of the singular includes the plural unless specifically stated otherwise. Also, the use of "comprise," "comprises," "comprising," "contain," "contains," "containing," "include," "includes," and "including" are not intended to be limiting. It is to be understood that both the foregoing general description and the following detailed description are exemplary and explanatory only and are not restrictive of the disclosure. The articles "a" and "an" are used herein to refer to one or to more than one (i.e., to at least one) of the grammatical object of the article. By way of example, "an element" means one element or more than one element.

Unless otherwise defined, all technical and scientific terms used herein have the same meaning as commonly understood by one of ordinary skill in the art to which this disclosure belongs. Methods and materials are described herein for use in the present disclosure; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

The details of one or more embodiments of the disclosure are set forth in the accompanying drawings and the description below. Other features, objects, and advantages of the disclosure will be apparent from the description and drawings, and from the claims.

### DESCRIPTION OF DRAWINGS

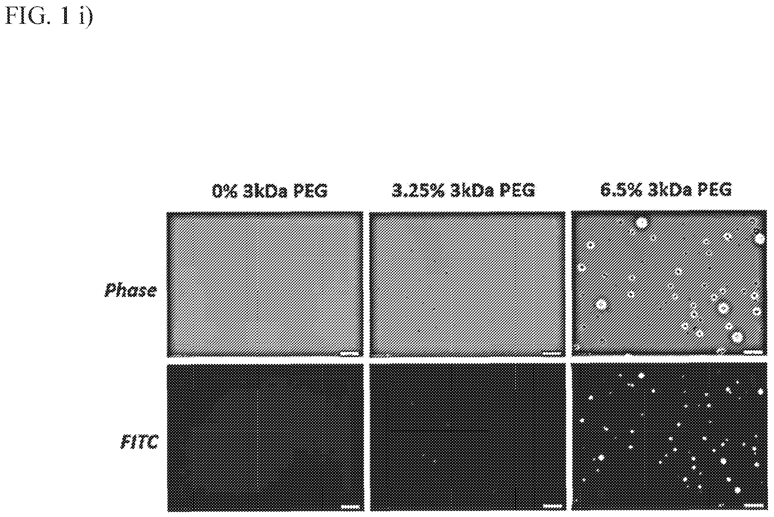

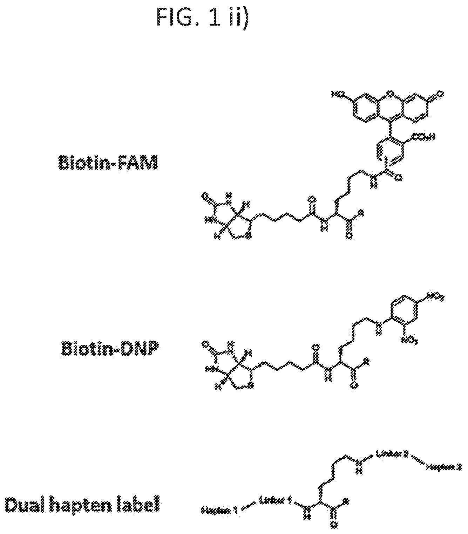

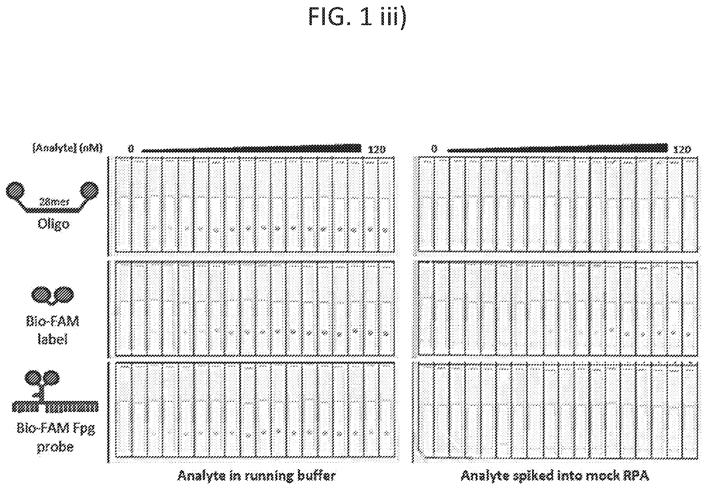

FIGs. 1 i)-iii) show an exemplary RPA reaction method using small dual-hapten analytes for undiluted detection on lateral flow strips. FIG. 1 i) is an image showing the formation of 3kDa PEG-induced coacervates and localization of FAM-labeled nucleic acids to the coacervates in mock RPA reactions. FIG. 1 ii) is a depiction of structures of exemplary dual-hapten analytes according to the present disclosure, biotin-FAM, biotin-DNP and generic label structures are shown, for post-synthetic attachment to amino-modified probe oligonucleotides. FIG. 1 iii) are images showing the detection of dual-labelled oligonucleotides, the Biotin-FAM dual label (R = OH) and Biotin-FAM Fpg probe diluted in running buffer or run undiluted in a mock RPA reaction.
FIGs. 2 i)-iii) depict direct analysis of RPA on lateral flow strips using dual-hapten Fpg probes according to the present disclosure. FIG. 2 i) is a schematic illustrating amplification-induced analyte release from RPA coacervates and subsequent detection on lateral flow strips. FIG. 2 ii) is an image of a prototype assay for undiluted RPA detection. FIG. 2 iii) is an image of dry conjugate format test strips incorporating flow control.
FIGs. 3 i)-iii) demonstrate the reduction of false positive signal through probe optimization. FIG. 3 i) shows images of an exemplary lateral flow assay where non-specific signal can be Fpg-dependent. FIG. 3 ii) is a depiction of hairpin and self-dimer structures of an exemplary Fpg dual-hapten probe (labeled `Probe 1' or `rs1207445'). FIG. 3 iii) shows images of amplifications containing an exemplary Fpg dual-hapten probe having reduced secondary structure exhibit reduced false positive signals when analyzed on lateral flow
FIG. 4 depicts images of undiluted RPA detection of rs 1207445 (genomic DNA) and *Campylobacter jejuni* (PCR product) template DNA.
FIGs. 5 i)-ii) demonstrate prototype *E. coli* O157:H7 serotype marker assays. FIG. 5 i) is TwistAmp Fpg fluorescence data for rfbEO157 assay. NTCs (red) and reactions containing 10 (yellow), 100 (green) and 1000 (blue) were performed in quadruplicate. Fluorescence reactions were compared to the prototype direct lateral flow assay. FIG. 5 ii) is a comparison of TwistAmp Fpg data for fliC_{H7} with the respective Fpg dual-hapten probe assay.
FIG 6. is an image demonstrating 'continuous flow' lateral flow strip analysis.
FIG. 7 are images of a lateral flow analysis of TwistAmp Nfo assay against the *Salmonella InvA* target
FIG. 8 is a schematic illustrating the mechanism for limited detection of dual-labelled amplicon when run undiluted on lateral flow strips.
FIG. 9 shows a schematic of undiluted Exo RPA LF detection.
FIG. 10 shows undiluted Fpg vs Exo RPALF.
FIG. 11 shows concurrent amplification/detection using Exo LF.
FIG. 12 show an exemplary continuous flow device.

### DETAILED DESCRIPTION

This disclosure is based, at least in part, on the discovery that RPA of a target nucleic acid sequence can be accurately and efficiently detected on a lateral flow strip with no dilution step, using a dual-hapten probe. To that end, provided herein are RPA compositions for detecting the presence or absence of a target nucleic acid using a dual-hapten probe. Also to that end, the present application discloses methods for detecting a target nucleic acid sequence on a lateral flow strip using a dual-hapten probe.

While, the present disclosure describe RPA compositions and methods for detecting a target nucleic acid sequence on a lateral flow strip using a dual-hapten probe, the skilled artisan would appreciate that the disclosed methods and compositions may be appropriate to other nucleic acid amplification methods (e.g., isothermal nucleic acid amplification methods) known in the field.

Rapid, cost-effective and sensitive detection of nucleic acids has the ability to improve upon current practices employed for pathogen detection in diagnosis of infectious disease and food testing. Furthermore, if assay complexity can be reduced, nucleic acid amplification tests could be deployed in resource-limited and home use scenarios.

A novel RPA Fpg (Formamidopyrimidine DNA glycosylase) probe chemistry has been developed, which allows lateral flow detection of amplification products in undiluted RPA reactions. To overcome the viscous nature of RPA reactions, a new type of dual-hapten label was developed for Fpg RPA probes. Exemplary assays were based on existing Fpg fluorescence assays (the rs1207445 human genomic locus and 16S rRNA of *Campylobacter jejuni*), which were modified for use with the new dual-hapten probe chemistry. The dual-hapten probe technology disclosed herein was then applied in the development of two novel singleplex assays for serotyping the genes of *E*. *coli* O157:H7 (*rjb_{O157}* and *fliC_{H7}*). These genetic markers are expected to identify forms of *E. coli* O157:H7 that other NAATs may miss, due to the complicated genetics of O157:H7. The aim was to develop a one-step, "sample in, results out" nucleic acid lateral flow immunoassay (NALFTA) and consumable for multiplex testing for O157:H7 for use in food hygiene testing. Furthermore, the versatility of the novel dual-hapten probe chemistry means that the technology could be readily applied to a wide array of target species permitting development of non-laboratory assays.

In the examples below, the novel nucleic acid lateral flow chemistry was applied to a human genomic target (rs1207445), *Campylobacter jejuni* 16S rDNA and two genetic markers of the important food pathogen *E. coli* O157:H7. All four assays have an analytical sensitivity between 10 and 100 copies DNA per amplification reaction. Furthermore, the assay requires fewer hands-on steps compared with existing RPA Nfo lateral flow assay methods.

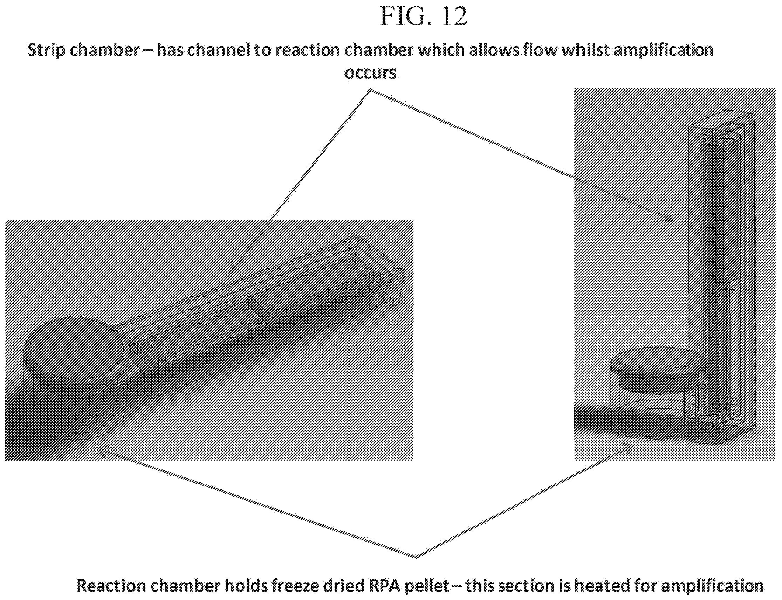

Data indicated that detection of amplified target nucleic acid can be performed concurrently with RPA ('continuous flow'). This allows the test time to be reduced (~30 minutes from sample to result). The simplified workflow, means the continuous flow chemistry could be readily adapted to a cost-effective single-use consumable, ideal for use in non-laboratory settings, such as point-of care (e.g., using the devices described in FIG. 12 or other devices).

In some cases, the dual-labeled oligonucleotide probes described herein comprises an oligonucleotide linked to a bifunctional structure (e.g., a dual-hapten leaving group). The bifunctional structure can include two moieties, e.g., two haptens, wherein one of the hapten is a first member of a first binding pair and the second hapten is a first member of a second binding pair. The probe is configured such that when the probe is bound to the target nucleic acid, the bifunctional structure is cleaved from the oligonucleotide, releasing the bifunctional structure. This free bifunctional structure (e.g., free dual-label) can then be detected by a number of methods, including, e.g., on a lateral flow strip.

"A member of a binding pair" is meant to be one of a first and a second moiety, wherein said first and said second moiety have a specific binding affinity for each other. Suitable binding pairs for use in the disclosure include, but are not limited to, antigens/antibodies (for example, digoxigenin/anti-digoxigenin, dinitrophenyl (DNP)/anti-DNP, dansyl-X-anti-dansyl, Fluorescein/anti-fluorescein, lucifer yellow/anti-lucifer yellow, peptide/anti-peptide, ligand/receptor and rhodamine/anti-rhodamine), biotin/avidin (or biotin/streptavidin) and calmodulin binding protein (CBP)/calmodulin. Other suitable binding pairs include polypeptides such as the FLAG-peptide (DYKDDDDK) [Hopp et al., BioTechnology, 6:1204 1210 (1988)]; the KT3 epitope peptide (Martin et al., Science 255:192 194 (1992)); tubulin epitope peptide (Skinner et al., J. Biol. Chem 266:15163 15166 (1991)); and the T7 gene 10 protein peptide tag (Lutz-Freyermuth et al., Proc. Natl. Acad. Sci. USA, 87:6393 6397 (1990)) and the antibodies each thereto. Generally, in a preferred embodiment, the smaller of the binding pair partners serves as the detectable label, as steric considerations may be important.

Nucleic acids (e.g., polynucleotides) suitable for amplification in connection with the present methods include double-stranded and single-stranded nucleic acid molecules, such as DNA and RNA molecules. The polynucleotides may be of genomic, chromosomal, plasmid, mitochondrial, cellular, and viral nucleic acid origin. For double stranded polynucleotides, the amplification may be of either one or both strands.

As described here, RPA employs enzymes, known as recombinases, that are capable of pairing oligonucleotide primers with homologous sequences in template double-stranded nucleic acid. In this way, DNA synthesis is directed to defined points in a template double-stranded nucleic acid. Using sequence-specific (e.g., gene-specific) primers, an exponential amplification reaction is initiated if the template nucleic acid is present. The reaction progresses rapidly and results in specific amplification of a sequence present within the template double-stranded nucleic acid from just a few copies of the template nucleic acid to detectable levels of the amplified products within minutes. RPA methods are disclosed, e.g., in US 7,270,981; US 7,399,590; US 7,666,598; US 7,435,561; US 2009/0029421; and WO 2010/141940, all of which are incorporated herein by reference.

The composition disclosed herein can contain a set of primers that amplify the target nucleic acid sequence. The primers can comprise of sequences that are complementary to the target nucleic acid sequence or that differ from the target nucleic acid sequence at one or more positions. As described herein, the amplification product, of RPA with a primer that differs from the target nucleic acid sequence at one or more positions, can differ from the target sequence at the one or more positions. The amplification product of the RPA reactions described herein can comprise a target sequence.

The set of primers can amplify the target nucleic acid sequence or they can introduce a sequence that differs from the target nucleic acid sequence at one or more positions. This introduced sequence can consist of a target nucleic acid sequence. The first primer can be complementary to the target nucleic acid sequence. The second primer can comprise a first portion that is complementary to the target nucleic acid sequence and a second portion that is different from the target nucleic acid sequence at one or more positions. When the two primers amplify the nucleic acid sequence the second primer incorporates the one or more different positions into the amplified products. This amplified region is different from the target nucleic acid sequence at the one or more positions and can consist of the target sequence. In some cases, the amplified region is the same as the target nucleic acid sequence.

The terms "first" and "second" are used in this disclosure in their relative sense only. It will be understood that, unless otherwise noted, those terms are used merely as a matter of convenience in the description of one or more of the embodiments. The terms "first" and "second" are only used to distinguish one element from another element, and the scope of the rights of the disclosed technology should not be limited by these terms. For example, a first element may be designated as a second element, and similarly the second element may be designated as the first element.

The RPA composition disclosed herein contains a recombinase, which may originate from prokaryotic, viral or eukaryotic origin. Exemplary recombinases include RecA and UvsX (e.g., a RecA protein or UvsX protein obtained from any species), and fragments or mutants thereof, and combinations thereof. The RecA and UvsX proteins can be obtained from any species. RecA and UvsX fragments or mutant proteins can also be produced using the available RecA and UvsS protein and nucleic acids sequences, and molecular biology techniques (see, e.g., the mutant forms of UvsX described in U.S. Patent No. 8,071,308). Exemplary UvsX proteins include those derived from myoviridae phages, such as T4, T2, T6, Rb69, Aehl, KVP40, *Acinetobacter* phage 133, *Aeromonas* phage 65, cyanophage P-SSM2, cyanophage PSSM4, cyanophage S-PM2, Rb14, Rb32, *Aeromonas* phage 25, *Vibrio* phage nt-1, phi-1, Rb16, Rb43, Phage 31, phage 44RR2.8t, Rb49, phage Rb3, and phage LZ2. Additional exemplary recombinase proteins include archaebacterial RADA and RADB proteins and eukaryotic (e.g., plant, mammal, and fungal) Rad51 proteins (e.g., RAD51, RAD51B, RAD51C, RAD51D, DMC1, XRCC2, XRCC3, and recA) (see, e.g., Lin et al., Proc. Natl. Acad. Sci. U.S.A. 103:10328-10333, 2006).

In any process of this disclosure, the recombinase (e.g., UvsX) may be a mutant or hybrid recombinase. In some embodiments, the mutant UvsX is an Rb69 UvsX that includes at least one mutation in the Rb69 UvsX amino acid sequence, wherein the mutation is selected from the group consisting of (a) an amino acid which is not histidine at position 64, a serine at position 64, the addition of one or more glutamic acid residues at the C-terminus, the addition of one or more aspartic acid residues at the C-terminus, and a combination thereof. In other embodiments, the mutant UvsX is a T6 UvsX having at least one mutation in the T6 UvsX amino acid sequence, wherein the mutation is selected from the group consisting of (a) an amino acid which is not histidine at position 66; (b) a serine at position 66; (c) the addition of one or more glutamic acid residues at the C-terminus; (d) the addition of one or more aspartic acid residues at the C-terminus; and (e) a combination thereof. Where a hybrid recombinase protein is used, the hybrid protein may, for example, be a UvsX protein that includes at least one region that includes an amino acid sequence derived from a different UvsX species. The region may be, for example, the DNA-binding loop-2 region of UvsX.

The DNA polymerase disclosed herein may be a eukaryotic or prokaryotic polymerase. Examples of eukaryotic polymerases include pol-alpha, pol-beta, pol-delta, pol-epsilon, and mutants or fragments thereof, or combinations thereof. Examples of prokaryotic polymerase include *E. coli* DNA polymerase I (e.g., Klenow fragment), bacteriophage T4 gp43 DNA polymerase, Bacillus stearothermophilus polymerase I large fragment, Phi-29 DNA polymerase, T7 DNA polymerase, Bacillus subtilis Pol I, Staphylococcus aureus Pol I, *E. coli* DNA polymerase I, *E. coli* DNA polymerase II, *E. coli* DNA polymerase III, *E. coli* DNA polymerase IV, *E. coli* DNA polymerase V, and mutants or fragments thereof, or combinations thereof. In some embodiments, the DNA polymerase lacks 3'-5' exonuclease activity. In some embodiments, the DNA polymerase has strand-displacing properties, e.g., large fragments of prokaryotic polymerases of class pol I or pol V.

In some embodiments, one or more probes (e.g., molecular beacon probes) are dual-labeled with detectable labels, which can be immunogenic. In some cases, the detectable labels are haptens. The two haptens on a probe can be the same or they can be different In some cases, one of the detectable labels is one member of a binding pair. The probes described herein can be labeled with haptens, enzymes, enzyme substrates, coenzymes, enzyme inhibitors, fluorophores, quenchers, chromophores, magnetic particles or beads, redox sensitive moieties (e.g., electrochemically active moieties), luminescent markers, radioisotopes (including radionucleotides), and members of binding pairs. More specific examples include fluorescein, phycobiliprotein, tetraethyl rhodamine, and beta-galactosidase. Binding pairs may include biotin/streptavidin, biotin/avidin, biotin/neutravidin, biotin/captavidin, epitope/antibody, protein A/immunoglobulin, protein G/immunoglobulin, protein L/immunoglobulin, GST/glutathione, His-tag/Metal (e.g., nickel, cobalt or copper), antigen/antibody, FLAG/M1 antibody, maltose binding protein/maltose, calmodulin binding protein/calmodulin, enzyme-enzyme substrate, receptor-ligand binding pairs, and analogs and mutants of the binding pairs.

As used herein, the term "hapten" refers to an immunogenic small molecule that reacts specifically with a binding partner, such as, for example an antibody generated against it. Haptens for use in the methods provided herein include, for example, digoxigenin, fluorescein, dinitrophenyl, glutathione, and biotin. Haptens described herein can also include, for example, an immunogenic group. In some cases, the immunogenic group comprises a fluorescent group, an enzyme or fragment thereof, a peptide or fragment thereof, or biotin. In some instances, the immunogenic groups are selected from the list comprising biotin, fluorescein, digoxigenin or dinitrophenyl.

As used herein, the terms "fluorescence label" and "fluorophore" are used interchangeably and refer to any substance that emits electromagnetic energy at a certain wavelength (emission wavelength) when the substance is illuminated by radiation of a different wavelength (excitation wavelength) and is intended to encompass a chemical or biochemical molecule or fragments thereof that is capable of interacting or reacting specifically with an analyte of interest in a sample to provide one or more optical signals.

Representative fluorophores for use in the methods provided herein include, for example, FAM, (tetramethylrhodamine) Texas Red^{™}, green fluorescent protein, blue fluorescent protein, red fluorescent protein, fluorescein, fluorescein 5-isothiocyanate (FITC), cyanine dyes (Cy3, Cy3.5, Cy5, Cy5.5, Cy7), Bodipy dyes (Invitrogen) and/or Alexa Fluor dyes (Invitrogen), dansyl, Dansyl Chloride (DNS-C1), 5-(iodoacetamida)fluorescein (5-IAF), 6- acryloyl-2-dimethylaminonaphthalene (acrylodan), 7-nitrobenzo-2-oxa-1,3,-diazol-4-yl chloride (NBD-Cl), ethidium bromide, Lucifer Yellow, rhodamine dyes (5-carboxyrhodamine 6G hydrochloride, Lissamine rhodamine B sulfonyl chloride, rhodamine-B-isothiocyanate (RITC), rhodamine 800); tetramethylrhodamine 5-(and 6-)isothiocyanate (TRITC)), Texas Red^{™}, sulfonyl chloride, naphthalamine sulfonic acids including but not limited to 1-anilinonaphthalene-8-sulfonic acid (ANS) and 6-(p-toluidinyl)naphthalen-e-2-sulfonic acid (TNS), Anthroyl fatty acid, DPH, Parinaric acid, TMA-DPH, Fluorenyl fatty acid, Fluorescein-phosphatidylethanolamine, Texas red-phosphatidylethanolamine, Pyrenyl-phophatidylcholine, Fluorenyl-phosphotidylcholine, Merocyanine 540, Naphtyl Styryl, 3,3'dipropylthiadicarbocyanine (diS-C3-(5)), 4-(p-dipentyl aminostyryl)-1-methylpyridinium (di-5-ASP), Cy-3 lodo Acetamide, Cy-5-N- Hydroxysuccinimide, Cy-7-Isothiocyanate, IR-125, Thiazole Orange, Azure B, Nile Blue, Al Phthalocyanine, Oxaxine 1,4',6-diamidino-2-phenylindole. (DAPI), Hoechst 33342, TOTO, Acridine Orange, Ethidium Homodimer, N(ethoxycarbonylmethyl)-6-methoxyquinolinium (MQAE), Fura-2, Calcium Green, Carboxy SNARF-6, BAPTA, coumarin, phytofiuors, Coronene, and metal-ligand complexes.

It should be noted that a fluorescence quencher is also considered a detectable label. For example, the fluorescence quencher may be contacted to a fluorescent dye and the amount of quenching is detected.

The embodiments described herein can also include an agent capable of cleaving a particular target nucleic acid sequence or a nuclease. As used herein, the term "nuclease" refers to enzymes capable of catalyzing the hydrolysis of nucleic acids, cleaving the phosphodiester bonds between the nucleotide subunits of nucleic acids. A "restriction nuclease" is a nuclease that targets and cleaves a nucleic acid molecule at or near specific recognition nucleotide sequences known as restriction sites. Nucleases may be further divided into endonucleases (i.e., enzymes that cleave the phosphodiester bond within a polynucleotide chain) and exonucleases (i.e., enzymes that work by cleaving nucleotides one at a time from the end (exo) of a polynucleotide chain), although some of the enzymes may fall in both categories. The nuclease can be a naturally occurring restriction endonuclease or an artificial endonuclease.

In some cases, the dual-hapten probes described herein are configured to be cleaved by formamidopyrimidine DNA glycosylase ("fpg")when the oligonucleotide probe is hybridized to complementary nucleotide sequence, to release the dual-hapten leaving group (e.g., the dual-label). In some cases, the nuclease is formamidopyrimidine DNA glycosylase.

In some embodiments, provided herein are hapten (e.g., dual hapten or higher order hapten) probes that are cleavable by an exonuclease (e.g., Exo III). In some embodiments, the oligonucleotide probe has the structure 5'X(n)ₐL(n)_{b}H(n)_{c}B3' wherein n are nucleotides a, b, and c are integers, X is a 5' hexyl or a hapten, H is a THF residue, B is a C3 spacer, and L is a branched modifier comprising a plurality of haptens. In some embodiments, the haptens are, for example DNP and Biotin, although other haptens may be utilized (e.g., FAM). In some embodiments, the oligonucleotide probe comprises a phosphorothioate link between the haptens. In some embodiments, a and c are 1 to 50 nucleotides and b is 0 to 50 nucleotides. In some embodiments, a and c are at least 15 nucleotides. In some embodiments, b is zero. In some embodiments, a is approximately 15 and c is approximately 30. In some embodiments, the oligonucleotide probe is complementary to target nucleic acid. In some embodiments, L replaces a cytosine nucleotide. Additional details of the probe are described in Example 16 below.

In an RPA reaction containing target amplicon, Exo III cleaves the abasic residue H of the probe, and subsequent 3'-5' digestion by Exo ill liberates a mononucleotide L (with 5'-phosphate and 3'-OH) that is labelled with two distinct haptens. This dual-hapten-labelled mononucleotide is free to exit RPA coacervates and interact with antibodies on visualising particles and the test line of a LF strip.

Additionally, one or more single-stranded DNA binding proteins can be used to stabilize nucleic acids during the various exchange reactions that are ongoing in the reaction. The one or more single-stranded DNA binding proteins can be derived or obtained from any species, e.g., from a prokaryotic, viral or eukaryotic species. Nonlimiting exemplary single-stranded DNA binding proteins include *E. coli* SSB and those derived from myoviridae phages, such as T4, T2, T6, Rb69, Aehl, KVP40, *Acinetobacter* phage 133, *Aeromonas* phage 65, cyanophage P-SSM2, cyanophage PSSM4, cyanophage S-PM2, Rb14, Rb32, *Aeromonas* phage 25, *Vibrio* phage nt-1, phi-1, Rb16, Rb43, Phage 31, phage 44RR2.8t, Rb49, phage Rb3, and phage LZ2. Additional examples of single-stranded DNA binding proteins include A. denitrificans Alide_2047, Burkholderia thailandensis BthaB_33951, Prevotella pallens HMPREF9144_0124, and eukaryotic single-stranded DNA binding protein replication protein A.

Any of the processes of this disclosure may be performed in the presence of a crowding agent. In some embodiments, the crowding agent may include one or more of polyethylene glycol, polyethylene oxide, polyvinyl alcohol, polystyrene, Ficoll, dextran, poly(vinylpyrrolidone) (PVP), and albumin. In some embodiments, the crowding agent has a molecular weight of less than 200,000 daltons. Further, the crowding agent may be present, e.g., in an amount of about 0.5% to about 15% weight to volume (w/v). In some cases, the crowding agent is PEG.

In some cases, the crowding agent has a molecular weight of at least 1 kilodalton, at least 2 kilodaltons, at least 3 kilodaltons, at least 4 kilodaltons, at least 5 kilodaltons, at least 6 kilodaltons, at least 8 kilodaltons, or at least 10 kilodaltons.

In some cases, the crowding agent is present in the composition at a concentration of at least 15% v/v, a concentration of at least 12% v/v, a concentration of at least 10% v/v, a concentration of at least 8% v/v, at least 6% v/v, a concentration of at least 5% v/v, a concentration of at least 4% v/v, or a concentration of at least 3% v/v.

In some cases, the crowding agent has a viscosity profile at 20 degrees Celsius of less than or equal to 5mPa/s, less than or equal to 4mPa/s, less than or equal to 3mPa/s, less than or equal to 2mPa/s, or less than or equal to 1mPa/s.

In some cases, the crowding agent is PEG with a molecular weight of 3 kilodaltons and a final concentration of 6.5%. In some cases, the crowding agent is PEG and the viscosity at 20 degrees Celsius is less than or equal to 3 mPa/s.

If a recombinase loading protein is used, the recombinase loading protein may be of prokaryotic, viral or eukaryotic origin. Exemplary recombinase loading proteins include *E. coli* RecO, *E*. *coli* RecR, UvsY, and mutants or fragments thereof, or combinations thereof. Exemplary UvsY proteins include those derived from myoviridae phages, such as T4, T2, T6, Rb69, Aehl, KVP40, *Acinetobacter* phage 133, *Aeromonas* phage 65, cyanophage P-SSM2, cyanophage PSSM4, cyanophage S-PM2, Rb14, Rb32, *Aeromonas* phage 25, *Vibrio* phage nt-1, phi-1, Rb16, Rb43, Phage 31, phage 44RR2.8t, Rb49, phage Rb3, and phage LZ2. In any of the processes of this disclosure, the recombinase loading agent may be derived from a myoviridae phage. The myoviridae phage may be, for example, T4, T2, T6, Rb69, Aehl, KVP40, *Acinetobacter* phage 133, *Aeromonas* phage 65, cyanophage P-SSM2, cyanophage PSSM4, cyanophage S-PM2, Rb 14, Rb32, *Aeromonas* phage 25, *Vibrio* phage nt-1, phi-1, Rb16, Rb43, Phage 31, phage 44RR2.8t, Rb49, phage Rb3, or phage LZ2.

Further, any of the processes of this disclosure may be performed with a blocked primer. A blocked primer is a primer which does not allow elongation with a polymerase. Where a blocked primer is used, an unblocking agent can be used to unblock the primer to allow elongation. The unblocking agent may be an endonuclease or exonuclease which can cleave the blocking group from the primer. Exemplary unblocking agents include *E. coli* exonuclease III and *E coli* endonuclease IV

The processes of this disclosure include the detection of a target nucleic acid sequence where the target nucleic acid may include a natural cut site for a restriction endonuclease or a nuclease. Additionally a cut site may be introduced into the target nucleic acid sequence by the amplification of the target sequence with primers that differ from the target nucleic acid sequence at one or more positions. The introduction of an artificial cut site or a cut site that was not found in the target nucleic acid sequence can be used to detect the target nucleic acid sequence or the presence of a SNP in the target nucleic acid sequence.

The processes described herein can also be performed in parallel using a variety of the restriction endonuclease or nucleases described herein. The detection of the amplification products can be performed in parallel and the rates of amplification compared to a reference sample. The processes described herein can be used for the detection of a target sequence, or the genotyping of a sequence.

In some of the embodiments, monitoring an increase of nucleic acid amplification products can include determining the number or proportion of amplification products in the reaction mixture over time or determining the number or proportion of dual label, e.g., dual-hapten or free dual-hapten/label.

In some embodiments, the dual-hapten is detectable by eye. In some embodiments, the dual-hapten label is detected using fluorescence, phase contrast microscopy, luminescent detection, spectral (color) detection, magnetic detection, radioisotopic detection and/or electrochemical detection. One of skill in the art would appreciate that any technique known in the art to measure the amount of nucleic acid amplification products in a mixture can be used to detect amplification products and monitor the increase in amplification products over time. In some of the RPA processes described herein a detectable label may be used to monitor the progress (the production of amplification products) of the RPA reaction.

The methods and compositions disclosed herein can be used, for example, to detect a target nucleic acid sequence. This disclosure can also identify a variant allele comprising a SNP compared to a wild type allele. In some cases, this SNP can be associated with a particular disease status or diagnosis (e.g., with the diagnosis of sickle cell anemia, or diagnosis of a tumor or cancer) or with a drug resistance or susceptibility. The isothermal amplification reaction methods and compositions described herein allow for the rapid detection of a target sequence and/or polymorphisms associated therein.

### EXAMPLES

### Example 1: Lateral flow strip materials and manufacture

Reagents and chemicals for buffer formulation were purchased from Fisher or Sigma.

Lateral flow strips were prepared using Prima 40 nitrocellulose (GE), adhesive backing cards (HF000MC100, Millipore) and CF5 absorbent pad material (GE) unless otherwise stated. For continuous flow experiments, additional wicking pad materials tested included CF6 (GE) and Grade 320 thick weight cellulose (Ahlstrom). Anti-DNP (MAB2223, Millipore) and anti-FAM (MIF2902, Thermo Fisher) monoclonal antibodies and anti-mouse polyclonal antibodies (A16162, Novex) were prepared for dispensing by exchanging the storage buffer to 10mM Sodium Phosphate pH 7.4 + 0.005% Triton X100 using Amicon Ultra 10k MWCO centrifugal concentrators, according to manufacturer's instructions. Purified antibodies were spotted onto pre-cut strips (0.5µg/strip) or dispensed onto membranes at a rate of 1µg/cm membrane using a Biodot ZX1010 dispensing platform. Membranes or dotted strips were dried for 1h at 40°C in a forced air oven prior to lamination.

Unless otherwise stated, strip laminates were prepared by lamination of 300mm × 25mm of anti-FAM/anti-DNP membrane flush with one long edge of the backing card (pre-cut to 300mm × 45mm). 300mm × 22mm strips of CF5 absorbent material were then laminated flush with the top edge of the backing card, such that the wicking pad overlaps the membrane by 2mm. Laminated cards were then cut to strips 5mm × 45mm using a Biodot CM5000 guillotine. Cut strips were stored at room temperature in a desiccator prior to use.

Where conjugate pads were employed, a gold conjugate was buffer exchanged by centrifugation for 20 minutes at 9000x g, the supernatant removed and the gold reconstituted to the original volume in 50mM Borax, 10% Sucrose, 1% Casein and 0.5% Brij-35. The conjugate was sprayed onto glass fibre strips (GFDX103000, Millipore) using the Biodot ZX1010 dispenser at a rate of 3µl/cm The gold conjugate pad was then dried for 2 hours at 40°C before lamination.

### Example 2: Conjugation of anti-biotin to 20nm gold colloid

Monoclonal anti-biotin (ab201341, Abcam) was prepared for conjugation using the AbPure BSA removal kit (Innova Biosciences) according to manufacturer's instructions. Purified anti-Biotin was conjugated to gold using the InnovaCoat Gold 20nm kit (Innova Biosciences) also according to manufacturer's instructions; with the exception that anti-biotin was present at 0.5mg/ml in the conjugation step. Anti-biotin gold was typically added direct to the RPA reaction, unless sprayed onto conjugate pads as described above.

### Example 3: Oligonucleotides and probe labelling

Oligonucleotide primers and unlabeled Fpg lateral flow probes were obtained from Eurogentec; unlabeled probes were purchased with an amino-modification at the abasic dR site, and a C3 spacer modification at the 3'-end. All fluorescent Fpg probes were obtained from LGC Biosearch.

### Example 4: Synthesis of dual-hapten labels and oligo labelling

Fmoc-Lys(Mtt)-Wang resin was purchased from Merck. A qualitative ninhydrin test kit was purchased from Anaspec. Dichloromethane (DCM), peptide synthesis grade *N,N-*dimethylformamide (DMF), (benzotriazol-1 -yloxy)tripyrrolidinophosphonium hexafluorophosphate (PyBOP), N-methylmorpholine (NMM), piperidine, D-biotin, triisopropylsilane (T1S), 5(6)-carboxyfluorescein, methanol, formic acid, acetonitrile, diethyl ether, triethylamine (TEA) for HPLC, acetic acid, NaHCO₃, NaOH, 2,4-dinitrofluorobenzene, and *N,N,N',N'*-tetramethyl-O-(N-succinimidyl)uronium tetrafluoroborate (TSTU) were purchased from Fisher Scientific. Trifluoroacetic acid (TFA), *N,N'*-dicyclohexylcarbodiimide (DCC), A-hydroxysuccinimide (NHS), *N*_{□}-DNP-L-Lys, 1,1,1,3,3,3-hexafluoro-2-propanol (HFIP), *N,N*-diisopropylethylamine (DiPEA), triethylamine (TEA) for LCMS, and water for LCMS were purchased from Sigma-Aldrich. Dimethylsulfoxide (DMSO) was from AppliChem. HCl and acetone were from VWR. Millipore water was used for synthesis and desalting; HPLC-grade water was used for HPLC purifications. NAP-10 size-exclusion (SE) columns were from GE. *N-*hydroxysuccinimidobiotin (biotin-OSu) was purchased from Iris Biotech.

LCMS analysis was performed on an Agilent 1200 LC system with an Agilent 6410B Triple Quadrupole ESI-MS. All LC methods employed an Agilent Eclipse Plus C18, 3.5 µm, 3.0 × 150 mm column at room temperature; all quoted yields are based on absorbance at 254 nm. LC solvents: A = water + 0.1% formic acid; B = acetonitrile + 0.1% formic acid; C = 200 mM HFIP, 4 mM TEA aq.; D = methanol. LC methods: 1 (solvents A and B) = 5 to 95% B over 25 min, then 95% B for 5 min; 2 (solvents C and D) = 20% D over 3 min, then 20 to 30% D over 5 min, then 30 to 50% D over 7 min, then 50 to 60% D over 5 min, then 60 to 80% D over 1 min, then 80% D over 1 min.

RP-HPLC purification of labelled oligonucleotides was performed on an Agilent 1100/1200 LC system equipped with a fraction collector, and a Phenomenex Clarity 10u Oligo-RP 250 × 4.6 mm column with an Oligo-RP guard cartridge (AJO-8135, Phenomenex). LC solvents A = methanol; B = 5% v/v acetonitrile in 50 mM TEAA pH 7.4. LC method for purification: 5% B over 5 min; 5 to 50% B over 35 min; 50 to 60% B over 5 min. Chromatograms were recorded at 254 nm and either 494 nm (for Bio-FAM labelling) or 360 nm (for Bio-DNP labelling).

Final labelled oligonucleotides were quantified using a Thermo-Fisher NanoDrop 2000 at 260 nm, assuming ε₂₆₀ = 20960 M⁻¹cm⁻¹ for the Bio-FAM label, and neglecting the ε₂₆₀ for the Bio-DNP label. NMR analysis was performed using an Oxford 400 MHz magnet equipped with a Bruker Avance console; d6-DMSO (99.9% atom D) was from Sigma-Aldrich.

### Example 5: Solid-phase synthesis of a Bio-FAM dual-hapten label

(D-)Bio-(L-)Lys(5(6)FAM)-OH was synthesized using standard solid-phase synthesis techniques (W. C. Chan and P. D. White, in Fmoc Solid Phase Peptide Synthesis, W. C. Chan and P. D. White, Oxford University Press, Oxford, 2000, ch. 3, pp. 41-76 ) starting from Fmoc-Lys(Mtt)-Wang resin. Specifically, resin (0.25g, 0.57 mmol/g loading) was swollen with DCM for 1 h, before a small sample was tested to confirm the absence of free amines (qualitative ninhydrin test). The Fmoc protecting group was removed using 20 % v/v piperidine in DMF (2 × 6 min), and the resin was washed with DMF (3 x) then DCM (3 x) before a qualitative ninhydrin test confirmed the presence of free amine. D-biotin (3 eq.) was activated with PyBOP (3 eq.) and NMM (5 eq.) in DMF (4.3 mL) for 4 min with sonication, before reaction with the resin at 60 °C for 38 min; the resin was subsequently washed with DMF (3 x) and DCM (3 x). Ninhydrin test of a sample of the resin confirmed the absence of free amines, before it was deprotected with 1 % v/v TFA, 5 % v/v TIS in DCM for 30 min (2 x), and subsequently washed with DMF (3 x) and DCM (3 x). Ninhydrin test of a sample of the resin showed free amine. 5(6)-Carboxyfluorescein (3 eq.) was activated with PyBOP (3 eq.) and NMM (5 eq.) in DMF (4.3 mL) for 10 sec, before reaction with the resin at 60 °C for 38 min; the resin was subsequently washed with DMF (3 x) and DCM (3 x). The resin was treated with 20 % v/v piperidine in DMF (2 × 10 min) to cleave fluorescein dimers, before the resin was washed with DMF (3 x), DCM (3 x), then MeOH (3 x); the washed resin was dried down and stored in the refrigerator (approx. 5 °C) overnight To obtain the free label, the resin was swollen in DCM (2 h), then approx. one-half of the swollen resin was cleaved with 2.5 mL of 90/2.5/2.5 v/v/v TFA/TIS/H₂O at room temperature for 2 h, washing with TFA (2 × 1 mL). Combined cleavage cocktails and TFA washes were dried under vacuum in a glass RB flask, then transferred to a 5 mL Eppendorf tube with DCM washings, blown down with Ar (g), and a yellow solid was precipitated upon addition of ice-cold diethyl ether (4.5 mL) and pelleted by centrifugation. The supernatant was discarded, and the pellet was washed centrifugally with diethyl ether (2 × 4.5 mL). Obtained 30 mg (58% yield) of a yellow solid. LCMS (method 1) of the product indicated good purity (90 %, tx 11.85 min); ESI-MS (pos. m/z): 731.3 (100%, [M+H]⁺). A 2.05 mM DMSO solution of the free label was prepared, which was further diluted with water for lateral flow studies employing the free Bio-FAM label.

### Example 6: Attachment of the Bio-FAM label to an amino-modified FPG probe

Bio-FAM dual label (5 mg, 6.8 µmol) was activated with DCC (2.1 mg, 10 µmol) and NHS (1.2 mg, 10 µmol) in the dark for 3 h, then a sample of the reaction mixture was diluted with methanol and analyzed by LCMS (method 1) which indicated the presence of the Bio-FAM NHS ester in 21% yield (*t*_{R} 12.97 min; ESI-MS (pos. m/z): 828.8 (9%, [M+H]⁺). After 3.5 h reaction time, 10 µ L of the activation mixture was added to a mixture of amino-modified probe oligo (rs1207445) at approx. 1 mM in water (20 µL) and 1 M pH 9 NaHCO₃ aq. (10 µL). The labelling mixture was vortexed, sonicated for 10 min, vortexed then left to stand at room temperature in the dark overnight The mixture was then desalted by NAP-10 SE column, purified by RP-HPLC (target ta 29.78 min), and the target fractions were concentrated *in vacuo,* desalted by NAP-10 SE, and concentrated further *in vacuo* to afford 190 µL of a 13.4 µM solution of the target Bio-FAM-labelled oligo (25 % yield). The labelled oligo was characterised by LCMS (method 2); 94 % purity, *t*_{R} 11.58 min, ESI-MS (neg.): requires 11584.4, found 11584.4. UV-Vis characterization (Thermo-Fisher NanoDrop 2000) revealed absorbance peaks at 259 and 496 nm, consistent with a FAM-labelled DNA oligo.

### Example 7: Solution-phase synthesis of a Bio-DNP dual-hapten label

(D-)Bio-(L-)Lys(DNP)-OH was synthesized using standard solid-phase synthesis techniques as described above for the Bio-FAM label using 2,4-dinitrofluorobenzene (3 eq.) and DiPEA (4 eq.)in DMF (4.3 mL) to incorporate the DNP moiety in place of FAM However, an improved, solution-phase synthesis was developed as described here. Specifically, Biotin-OSu (602 µmol, 1.05 eq.) and Nu-DNP-L-Lys (1 eq.) were suspended in DMF (5.7 mL) and DiPEA (0.25 mL), and sonicated for 30 min to obtain a clear solution before stirring at room temperature. After 2 h, a precipitate was observed in the reaction mixture, which was left to stir at room temperature overnight before filtration (through 2 × Whatman No. 1 filter papers), washing with acetone (10 mL) and diethyl ether (130 mL) to collect a yellow solid (244 mg). A second crop of yellow solid (30 mg) was obtained by combining the organic washes, and adding additional diethyl ether (100 mL) before filtration as above and washing with further diethyl ether (100 mL). Both crops of the DiPEA salt of the target label were combined and dissolved in 0.5 M NaOH aq. (5 mL) to afford a deep orange-yellow solution, before 1 M HCl aq. (5 mL) was added to precipitate the carboxylic acid target as a yellow solid. The mixture was cooled on ice before the solid was collected by suction filtration (through 2 × Whatman No. 1 filter papers), washing with ice-cold 1 M HCl aq. (2 × 25 mL), then H₂O (2x 25 mL), then diethyl ether (650 mL). The washes were discarded, and the yellow solid was dried *in vacuo* to obtain the target in good yield (0.41 mmol, 72%). LCMS (method 1) 97 % purity, *t*_{R} 13.71 min, ESI-MS (pos. m/z): 539.2 (18%, [M+H]⁺).; 1H NMR (400 MHz, d6-DMSO) δ_{H} 12.51 (br s, 1 H, OH), 8.88 (d, 1H, *J =* 5.8 Hz, NH-Ar), 8.86 (d, 1H, *J =* 2.7 Hz, Ar-H3), 8.25 (dd, 1H, *J =* 9.7, 2.6 Hz, Ar-H5), 8.05 (d, 1H, *J =* 7.8 Hz, NHC(O)CH₂), 7.22 (d, 1H, *J =* 9.7 Hz, Ar-H6), 6.38 (d, 2H, *J* = 16.2 Hz, NHC(O)NH), 4.29 (dd, 1H, *J =* 7.5, 5.0 Hz, CH(CH₂)S), 4.20-4.10 (m, 2H, CHCO₂H, NHCHCH(R)S), 3.52-3.43 (m, 2H, CH₂NHAr), 3.10-3.06 (m, 1H, NHCHCH(R)S), 2.80 (dd, 1H, *J=* 12.4, 5.0 Hz, CH(H)S), 2.56 (d, 1H, *J* = 12.4 Hz, CH(H)S), 2.11 (t, 2H, NHC(O)CH₂), 1.78-1.25 (m, 12H, 6 × CH₂).

### Example 8: Attachment of the Bio-DNP label to an amino-modified FPG probe

Bio-DNP dual label (10 mg, 18.6 µmol) was activated with TSTU (8.4 mg, 27.9 µmol) and DiPEA (9.7 µL, 27.9 µmol) for 8 min, then a sample of the reaction mixture was diluted with acetonitrile and analyzed by LCMS (method 1) which indicated the presence of the Bio-DNP NHS ester in 19% yield (*t*_{R} 15.09 min; ESI-MS (pos. m/z): 636.3 (9%, [M+H]⁺). After 1.5 h reaction time, 10 µL of the activation mixture was added to a mixture of amino-modified probe oligo (rs1207445) at approx. 1 mM in water (20 µL) and 1 M pH 9 NaHCO₃ aq. (10 µL). The labelling mixture was vortexed, sonicated for 10 min, then left to stand at room temperature in the dark overnight. The mixture was then desalted by NAP-10 SE column, purified by RP-HPLC (target *t*_{R} 31.10 min), and the target fractions were concentrated *in vacuo,* desalted by NAP-10 SE, and concentrated further *in vacuo* to afford 470 µL of a 7.1 µM solution of the target Bio-DNP-labelled oligo (17% yield). The labelled oligo was characterised by LCMS (method 2); 90 % purity, *t*_{R} 10.48 min, ESI-MS (neg.): requires 11392.4, found 11392.2.

### Example 9: RPA conditions

All RPA reactions were incubated at 40°C for 20 minutes unless stated otherwise. RPA formulations for lateral flow (Fpg) contain 420nM each of the appropriate forward and reverse primer, 120nM of the dual-hapten Fpg probe, 50mM Tris Acetate pH 8.3, 100mM KOAc, 5mMDTT, 1× creatine kinase, 30µg Gp32, 30µg UvsX, 7µg UvsY, 6.5% 3kDa PEG, 5.7% trehalose, 8.6µg DNA polymerase I (*S*. *aureus*), 9.48µg Fpg, 1× E-mix, 1.8mM dNTPs and 0.5% Brij-35. Reactions are initiated by the addition of a mix containing the appropriate template and Mg(OAc)₂ (22.5mM final concentration) to a final volume of 100µl.

Nfo RPA reaction formulations were the same as Fpg, with the exception of Gp32 (28µg/reaction) and polymerase (12.8µg/reaction). Additionally, Fpg is replaced with Nfo (Endonuclease IV; 4.6pg/reaction).

Fluorescent Fpg reactions were performed using commercially available TwistAmp Fpg kits according to manufacturer's instructions on a T8 instrument (Axxin). Reactions were incubated for 20 minutes at 40°C.

### Example 10: Lateral flow strip detection

Unless otherwise stated, 1.5µl anti-biotin gold was added directly to the completed RPA reaction and the lateral flow strip added Strips were allowed to wick for 20 minutes before drying at room temperature for 10 minutes. Absorbent pads and glass fibre conjugate pads (where appropriate) were removed and the strips scanned.

Diluted detection on PCRD strips (Abingdon Health) was performed by dilution of 5µl amplicon into 70µl PCRD extraction buffer. The entire 75µl was processed on the PCRD device according to manufacturer's instructions.

### Example 11: Undiluted detection of novel analytes on lateral flow strips

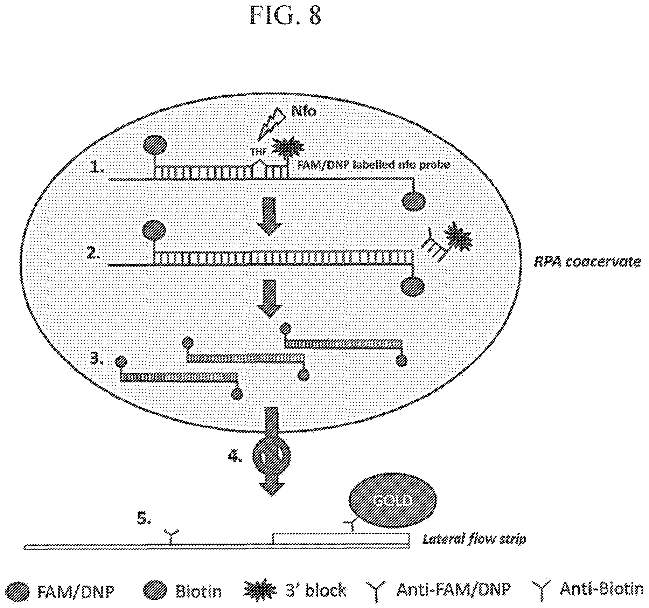

To date, detection of RPA products on lateral flow devices has been achieved using commercially available NALFIA strips, such as for example Milenia Hybridetect strips, Abingdon Health PCRD strips and UStar devices in combination with the TwistAmp Nfo chemistry, which generates a dual-hapten labelled amplicon that comprises each label attached to single stranded nucleic acid, which hybridise to form the dual-hapten label that can be detected by sandwich lateral flow immunoassay. Whilst this technology performs comparably to fluorescent probe based approaches in terms of sensitivity and specificity, end users must first dilute the highly viscous amplicon in order to permit migration of the label along the assay strip and therefore permit successful determination of amplification products on such lateral flow strips (FIG. 8). A schematic illustrating the mechanism for limited detection of dual-labelled amplicon when run undiluted on lateral flow strips is shown in FIG. 8. The TwistAmp Nfo reactions contain a 3' blocked, hapten-labelled probe (containing an internal tetrahydrofuran (THF) residue), a hapten-labelled reverse primer and Endonuclease IV (Nfo). In this study, probes were typically labelled with biotin, whilst the reverse primer was labelled with either DNP or FAM. Upon amplification, the biotin-labelled probe binds to the newly-synthesised, DNP/FAM-labelled strand. Once the probe has bound, Nfo cleaves the phosphodiester bond 5' of the THF residue (Step 1). The cut probe acts as a primer for a strand displacing polymerase which extends the probe effectively removing the 3' block (Step 2). Successive rounds of RPA therefore result in the generation of dual-hapten labelled amplicons (Step 3). However, we believe that unless the reaction is diluted, the majority of the analyte remains sequestered in RPA coacervates (Step 4), rendering it largely unavailable to bind at the test line resulting in false negative/weak true positive test line signals.

The present disclosure provides means for end users to perform lateral flow determinations with a reduced number of hands-on steps, in particular with no dilution steps being required for successful lateral flow separation directly from the RPA amplicon mixture (direct detection).

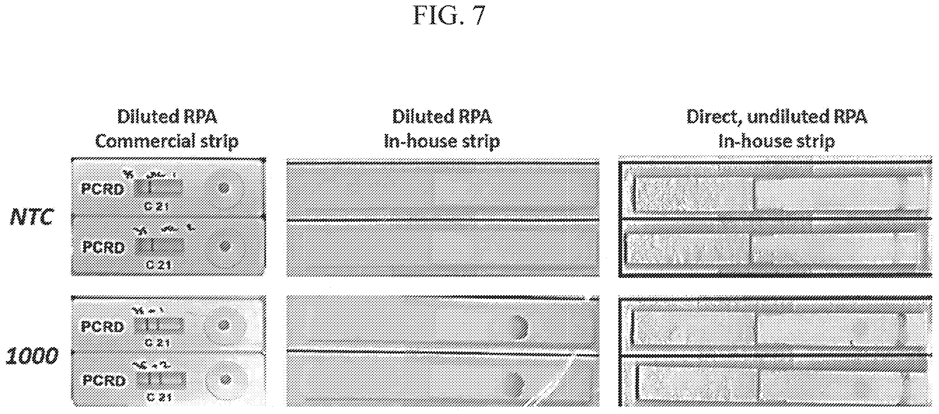

Initially the direct analysis of RPA on lateral flow devices without dilution proved unsuccessful. The high concentration of 35kDa PEG used in the standard RPA amplification mixture meant that reactions were too viscous to wick fully, and a significant portion of the gold colloid aggregated at the proximal end the strip. The present disclosure provides an improved RPA mixture, which is designed to mitigate the effects observed when using 35 kDa PEG. The modified RPA mixture utilises low molecular weight PEG as the crowding agent (6.5% 3kDa PEG) and 0.5% v/v Brij-35, which greatly improves the migration of gold colloid through the nitrocellulose membrane. Although commercially available TwistAmp Nfo chemistry (which comprises 35kDa PEG) permitted some detection of amplicons once they were suitably diluted with running buffer on existing lateral flow assay strips produced commercially or in-house, there was little stimulation in detectable signal at the test spot in RPA reactions containing 1,000 copies of template vs. NTCs when low MW PEG (6.5% 3kDa PEG) Nfo RPA is run undiluted on strips (FIG. 7).

Lateral flow analysis of TwistAmp Nfo assay against the *Salmonella InvA* target is shown in FIG. 7. TwistAmp Nfo reactions (NTCs and 1,000 copies template DNA per reaction; TwistAmp Nfo reactions contain 35kDa PEG). RPAs were analysed diluted 1/50 in running buffer on commercial lateral flow strips (PCRD, left panel) and anti-FAM strips produced in-house (middle panel). In parallel, 3kDa PEG RPA reactions were analysed undiluted on the same batch of anti-FAM strips (right panel).

To determine whether a dual-hapten label would be more applicable to direct, undiluted detection on lateral flow strips, three types of analyte were spiked into TBST (from 0 to 120nM) or into mock RPA reactions containing all RPA components in 6.5% 3kDa PEG, prior to analysis on strips. The three analytes were: 1) A 28mer oligonucleotide labeled at the 5' and 3' ends with FAM and Biotin respectively; used to simulate a hapten labeled amplicon; 2) The novel Bio-FAM dual-hapten probe; 3) An Fpg probe, where the abasic dR group is labeled with the Bio-FAM dual-hapten. Importantly, all three analytes were detected to a similar extent when spiked into buffer, with positive signals observed to the lowest concentration of analyte tested (~1nM). When detections were performed undiluted against spiked mock RPA, strips that were run in the absence of analyte demonstrated some weak non-specific signal. Weak signals were also observed up to 120nM analyte for the dual-labeled oligonucleotide and the dual-hapten Fpg probe. However, significant stimulation in the test line signal vs. negative samples was only observed for the free dual-hapten analyte (FIG. 1 iii). These data agree with the hypothesis that small labels could be used for undiluted detection of RPA products on lateral flow strips, whereas labeled nucleic acids labels (as liberated from existing commercial Nfo RPA reactions) are not readily detectable, because of the localisation of such bulky probes within RPA coacervates, which limits availability for detection of such labels on the lateral flow strip.

### Example 12: Direct detection of RPA on lateral flow strips

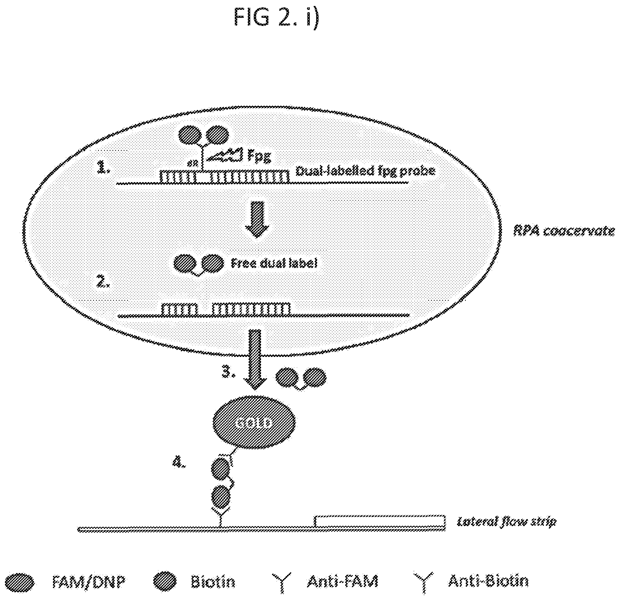

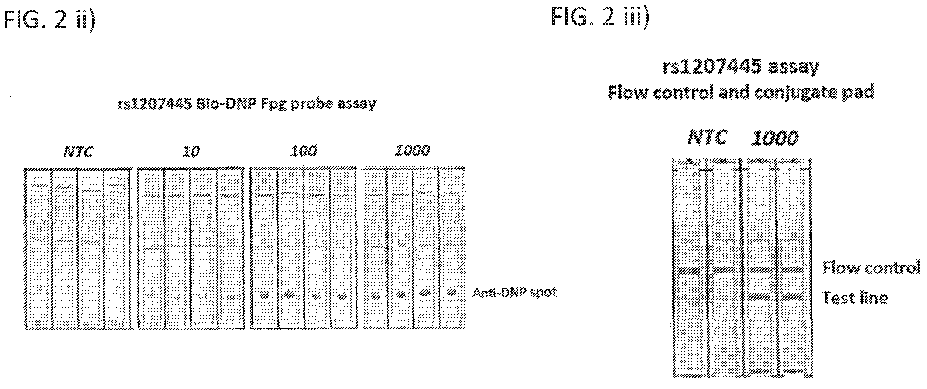

In order to use the dual-hapten analyte for detection of RPA, it is required that the label is effectively sequestered until amplification occurs. Therefore, an Fpg probe against the rs1207445 target was purchased with an internal amino-modified dR abasic site, which can be conjugated to the dual-hapten label (rs1207445 Probe 1). During RPA, the probe hybridises to the amplicon, at which point Fpg cleaves the abasic site at the 5' and 3' of the dR group by βδ elimination (FIG. 2 i), Step 1), releasing the dual-hapten label (FIG. 2 i), Step 2). Due to its small size, the dual-hapten analyte is free to escape from RPA coacervates (FIG. 2 i), Step 3), such that it can be detected by sandwich immunoassay on the lateral flow strip (FIG. 2 i), Step 4). Importantly, unprocessed probe (which could theoretically be detectable as it is coupled to both haptens) is likely to be sequestered in RPA coacervates, which render it largely undetectable in RPA reactions where no amplification takes place. This effect is exemplified by the fact that neat probe spiked into mock RPA reactions is less detectable than the free label (FIG 1 iii).

To obtain proof-of-principle that the Fpg dual-hapten probes could be used for direct endpoint detection of RPA on lateral flow, low MW PEG RPA reactions against 10, 100 and 1,000 copies/reaction of human gDNA (plus NTCs) containing the rs1207445 dual-hapten probe (Probe 1) were performed in quadruplicate. 1.5µl anti-biotin gold was added and the strip dropped into the amplicon/gold mix. Some non-specific signal was observed in NTC reactions, but minor stimulation of the test line signal was observed in 2/4 replicates of RPA containing 10 copies of template. Strong stimulation of test line signal vs. NTCs was observed in all reactions containing ≥ 100 copies gDNA per reaction (FIG. 2 ii). These preliminary data indicate the new Fpg probe chemistry permits undiluted detection of RPA products on lateral flow strips.

Many commercial NALFIA strips incorporate the detection conjugate onto the strip, by drying the colloid into a conjugate pad. Furthermore, most strips also include a flow control line, which acts to confirm that strips have wicked effectively. To determine if strips could be produced for direct detection of RPA that incorporate these features, strips were lined with an anti-DNP test line, an anti-Mouse flow control line (which binds any conjugate that bypasses the test line) and a glass fibre conjugate pad containing dried anti-Biotin gold colloid. The rs1207445 assay was then performed in the absence or presence of 1000 copies of human gDNA, before dropping the strips directly into the RPA once the reaction had completed. In all cases, effective flow was observed as evidenced by the strong signal at the flow control line. Similar to that observed previously, some weak non-specific signal was observed at the anti-DNP test line in negative amplifications, however, significant stimulation in the test line signal was observed in positive amplifications versus those containing no template (FIG. 2 iii).

Taken together, these data indicate that the new dual-hapten Fpg probe can be used for direct, undiluted detection of RPA products on lateral flow strips.

### Example 13: Effect of probe design on the false positive signal in negative RPA reactions

Non-specific signals may be observed by eye in some negative reactions; however, if one were to make use of a digital reader (such devices are routinely used with commercial lateral flow assays), the instrumentation can be configured to subtract background signals through image analysis algorithms. If assay test strips are to be used in resource-limited settings, it may be necessary to visualise the assay strips by eye, in which case the ability to minimise non-specific signals at the test line is desirable to mitigate possible user misinterpretation of results.

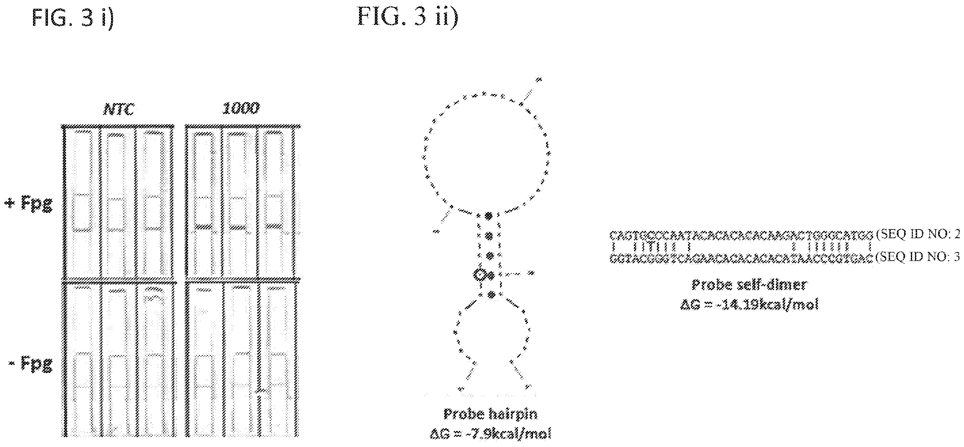

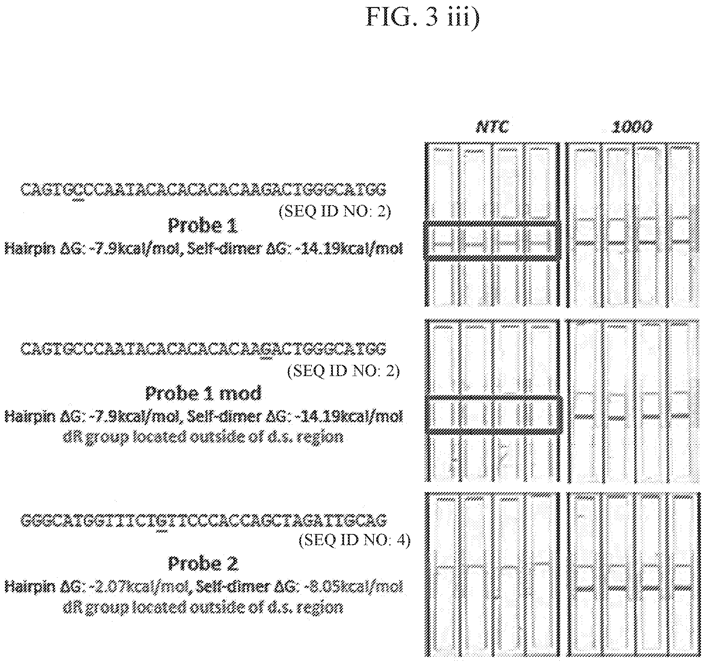

The fact that non-specific signal is observed in negative reactions is not necessarily an issue if the lateral flow strip is analysed using a strip reader, as this can subtract the background signal in any analysis. However, if the strips are to be used in resource-poor settings, it is desirable to be able to visualise the strips by eye, in which case the non-specific signal at the test line is less desirable. Therefore, the cause of the false positive signal was determined by performing low MW PEG Fpg RPA reactions for rs1207445 in the presence or absence of Fpg, to determine whether the noise was due to the oligonucleotide probe binding directly to the test line or as a result of aberrant probe processing by the Fpg enzyme. In the presence of Fpg, rs1207445 RPA demonstrated false positive signal in NTC reactions, with a stimulation in test line signal in reactions containing 1,000 copies of human genomic DNA template. The reactions performed in the absence of Fpg demonstrated significantly reduced non-specific signal in NTC reactions (and no amplification in reactions containing 1,000 copies of template DNA), suggesting that the rs1207445 dual-hapten probe was being aberrantly processed leading to the false positive signal (Figure 3i). In some cases some non-specific signal remains in the absence of Fpg and this signal can be effectively removed by blocking of the membrane.

To determine the cause of the Fpg-dependent non-specific signal, the rs1207445 dual-hapten Fpg probe sequence was analysed for hairpins, primer/probe and probe/probe dimers using NetPrimer (available at http://www.premierbiosoft.com/netprimer/) or the OligoAnalyser 3.1 program (IDT; available at https://eu.idtdna.com/calc/analyzer). These analyses revealed that the probe could form a weak hairpin structure (ΔG = -7.9kcal/mol) and a strong self-dimer (ΔG= -14. 19kcal/mol). In both cases, the dR group (highlighted in red in Figure 3ii) lies in a double-stranded context and can therefore act as a substrate for the Fpg enzyme, providing a potential explanation for why there is Fpg-dependent noise in NTC RPA reactions.

Two new rs1207445 probes were designed: 1) Probe 1 mod - this probe has the same sequence as Probe 1 and forms the same stuctures. However, the abasic site is now located outside of the putative double-stranded regions and should not be processed by Fpg; 2) Probe 2 - this dual-hapten probe is designed to hybridise to a different region within the rs1207445 amplicon. This probe demonstrates reduced hairpin (ΔG = - 2.07kcal/mol) and self-dimer formation (ΔG = -8.05kcal/mol) vs. Probe 1 and Probe 1 mod. Furthermore, the dR group is located outside of any double-stranded context and should not serve as a substrate for Fpg (Figures 3 ii and iii).

To determine whether the improved probe designs could reduce the non-specific signal in negative amplifications whilst maintaining the ability to detect RPA in positive amplifications, NTC and positive (containing 1000 copies of human gDNA) RPA reactions in the presence of the three probes were detected undiluted on lateral flow strips. All positive reactions demonstrated significant stimulation in test line signal over negative amplifications, suggesting that RPA was performing as expected (Figure 3iii). rs1207445 Probe 1 demonstrated the highest background signal in negative amplifications. The non-specific signal was barely detectable in negative amplifications containing Probe 1 mod, and was not visible in reactions containing Probe 2 (Figure 3iii). Thus, the non-specific signal in negative amplifications can be effectively reduced or eliminated by designing probes which do not form or reduce the likelihood of forming secondary structures, self- or cross-dimers. In some cases these structures cannot be avoided, and the abasic site should be located outside of any double-stranded context.

### Example 14: Direct detection of RPA on lateral flow - analytical sensitivity and applicability to other targets

Further exemplification of the Fpg dual-hapten probe for direct detection of RPA was performed. The rs1207445 assay and another existing in-house assay for *Campylobacter jejuni* were initially compared. The *C. jejuni* assay utilised previously designed primers targeted against the 16S rRNA, with the only modification being that the existing Fpg probe sequence was replaced with the new dual-hapten labeled probe designed for direct lateral flow detection.

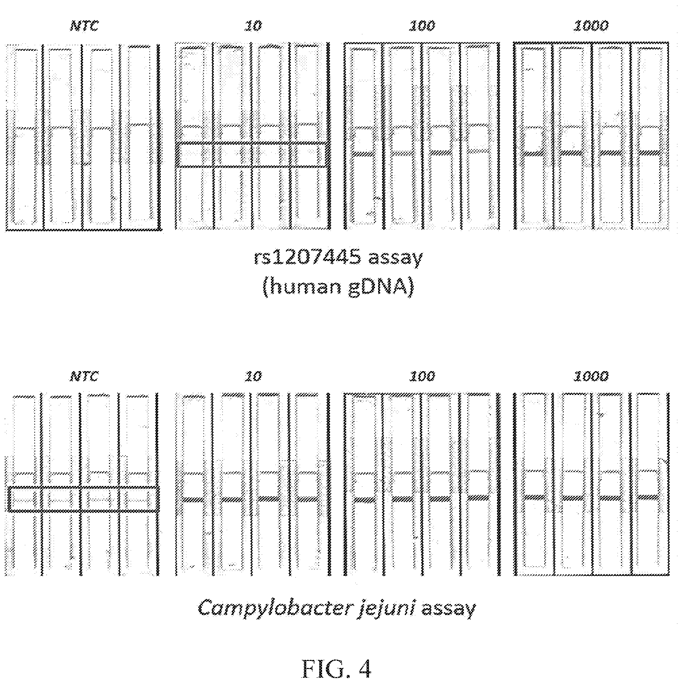

In both examples, low MW PEG RPA was performed in quadruplicate, with reactions containing 10, 100 or 1,000 copies of the appropriate template DNA (human gDNA and synthetic 16S *C*. *jejuni* rDNA template for rs1207445 and Campylobacter assays respectively). RPA products were then analysed undiluted on anti-DNP lateral flow strips. In the improved rs1207445 assay (utilising rs1207445 Probe 2), no signal was observed at the test line in NTC reactions. Test lines were weak but present in all RPA containing 10 copies of template DNA (outlined in green on FIG. 4 i); more visible at the strip edge), with good signals observed on test strips for all RPA reactions containing ≥100 copies of human gDNA (FIG. 4 i).

In the *C*. *jejuni* assay there was a weak false positive signal present in all NTCs (it should be noted that this probe was not redesigned for lateral flow, it exhibits some minor secondary structure and self-dimerization). However, very strong test line signals were observed in all amplifications containing ≥ 10 copies of template DNA (FIG. 4 ii).

Taken together, these data suggest that lateral flow strip detection is limited only by the success of the amplification reaction; the same improved RPA chemistry for use with direct detection lateral flow assay was used for both cases. Furthermore, the data demonstrate the improved Fpg probe chemistry may be applied to a wide-range of RPA processes for detection of target nucleic acid. Such improved technology could be readily applied to many potential targets, with analytical sensitivities expected to be equivalent to other commercial NAAT assay formats. Although some false positive signals were observed in the *C*. *jejuni* assay, it is expected such observations could be mitigated through a process of careful selection of probe nucleotide sequence to minimise possibility for the formation of self-dimers/secondary structures; as was observed with the revised probe used in the assay for rs1207445.

Further exemplification of the improved direct detection lateral flow technology was performed Novel RPA primers and probes for the detection of *E. coli* O157:H7 were prepared. Such an assay may be used for food testing, with the potential for use in rapid diagnosis of disease using faecal samples. RPA lateral flow assays were developed that could be used to identify the serotype marker genes (*rjb_{O157}*) and (*fliC_{H7}*) which represent highly conserved regions within in all strains of *E. coli* O157:H7. The newly developed RPA assays were initially designed as singleplex tests, with scope for biochemical multiplexing of the test at a future date (using Fpg probes labeled with Bio-DNP and Bio-FAM dual-hapten labels respectively, since such probes may be independently detected at different capture lines on a lateral flow test strip).

Preliminary screening of all primer/probe combinations for the *E. coli* serotypes was performed using TwistAmp Fpg fluorescent probe assays (containing 5.5% 35kDa PEG) using the isothermal T8 instrument with the intention of discovering primer/probe combinations that exhibited an analytical sensitivity of ~10 copies per reaction, with rapid amplification kinetics (onset times of <6 minutes at 10 copies, with high maximum fluorescence). The best performing probes in fluorescent assays were then modified for use as dual-hapten Fpg probes in low MW PEG RPA for use in direct assay lateral flow (the primers are the same between fluorescent and lateral flow assays).

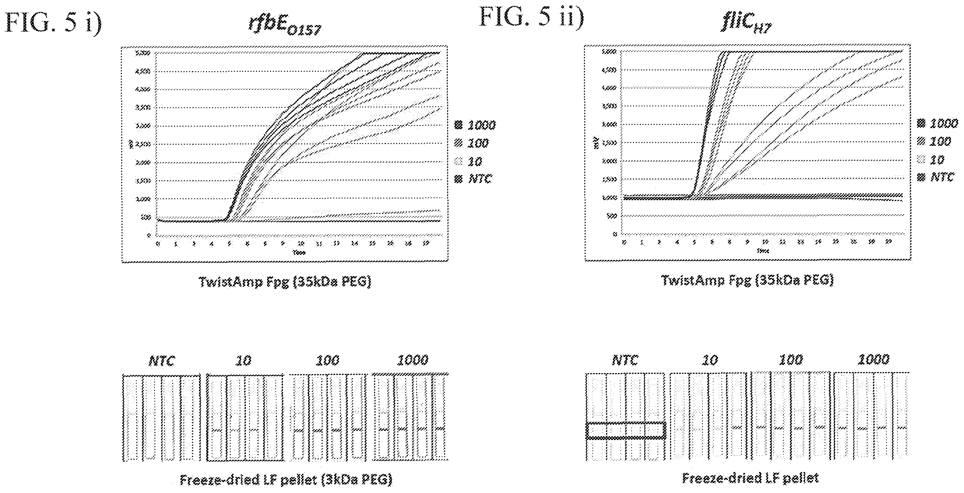

FIG. 5 i) shows a comparison of the novel *rjb_{O157}* fluorescent Fpg assay (35kDa PEG) against the assay for direct, undiluted detection of low MW PEG RPA reactions on lateral flow strips. The fluorescent probe assay (using 35kDa PEG) exhibits signal above the NTC baseline (in red) in all replicates at 10 copies of template DNA (quantified synthetic DNA). As expected, all reactions containing 100 copies (green) and 1,000 copies (blue) were positive, with onset times and maximal fluorescence correlating well with the amount of template DNA per reaction. In the direct lateral flow assay, positive test line signals were observed in three of four replicates at 10 copies of template DNA (all replicates containing ≥100 copies of template were strongly positive). No visible false positive signal was observed in NTCs, further demonstrating that good probe design can eliminate such artefacts (because the same strip chemistry as used for the rs1207445 assay was also utilised in detection of *E*. *coli*)*.*

FIG. 5 ii) shows the performance of the *fliC_{H7}* fluorescent probe singleplex as compared to the direct lateral flow method. Again, the fluorescent probe assay showed strong amplification to 10 copies (yellow) in all replicates tested, with maximal fluorescence and onset times correlating well with the amount of copies of quantified synthetic DNA template present in the reaction. In the Fpg dual-hapten lateral flow assay, a weak signal was observed at 10 copies in three of the four replicates, with a stronger signal for one replicate. A very weak false positive signal was observed in all NTCs for the *fliC_{H7}* assay; however; such nonspecific signal could be eliminated through further iterative probe design and the use of blocking reagents, as previously demonstrated.

These data demonstrate the versatility of the novel dual-hapten Fpg probe chemistry, and show that sensitive assays can be designed against important pathogens with relative ease, achieving comparable assay sensitivity compared to existing commercial fluorescence Fpg assays.

### Example 15: Improved ease-of-use of the Fpg dual-hapten lateral flow assay - 'Continuous flow'

Direct, undiluted detection of RPA products can reduce the complexity of a assay consumables, thereby reducing manufacturing costs whilst making the test procedure simpler for end-users. The present disclosure may reduce test times from approximately 40 minutes using the Fpg dual-hapten lateral flow RPA assay to 30 minutes or less depending on the required assay sensitivity.

Feasibility studies were performed to demonstrate to the benefits of having the lateral flow assay strip present in the RPA reaction throughout the amplification cycle. Reaction volumes were increased from 100µl to 200µl to accommodate the fact that the mixture starts migrating along the strip before significant amplification has occurred. Initially, anti-Biotin gold colloid label was placed directly into the RPA reaction mix before amplification to prevent the release of conjugate from the pad before amplicon had accumulated. Different heavy weight wicking pad materials (Control material: CF5, medium weight cellulose fibre; Experimental materials:CF6 and Grade 320, heavy weight celluloses) were evaluated, in order to increase the volume of reaction that could be processed on the strip. Assay strips were laminated with an adhesive cover tape, to help prevent the wicking pad from delaminating from the device as the RPA assay proceeds.

Proof-of-concept was established using the rs1207445 Fpg dual-hapten (Bio-DNP) assay, using 200µl freeze-dried RPA pellets for use with the lateral flow assay (3kDa PEG). Pellets were hydrated with a buffer container containing either 0 or 5000 copies of template DNA plus 1.5µl anti-Biotin gold colloid, and anti-DNP strips with wicks made of CF5 (control), CF6 or grade 320 heavyweight pads; all reaction mixtures were added immediately to each of the lateral flow strips, which were incubated at 40°C for 30 minutes.

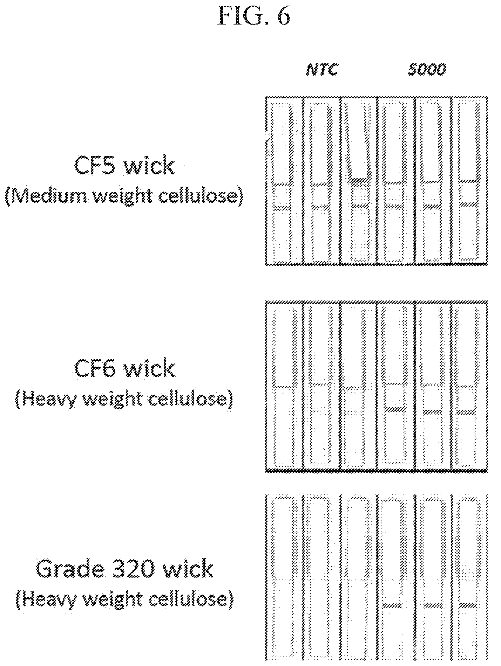

In FIG. 6, strips made with the indicated absorbent pad materials were incubated in the RPA reaction during amplification. Data shown is for the rs1207445 dual-hapten Fpg assay, NTC reactions were compared to those containing 5000 copies of template DNA.

In the control (CF5) strips, a very strong false positive signal was observed in all NTC reactions, with only minor stimulation of the test line signal at 5000 copies of input template DNA (FIG. 6 i), top panel). However, whilst the CF6 strips gave some false positive signal (weaker than in control strips), the false positive response was effectively eliminated when using Grade 320 strips. Which is expected to result from the large bed volume of the Grade 320 material, which may have improved capillary action, compared with the CF5 or CF6 materials. Analyte may flow more readily through the Grade 320 material based strips compared to the CF5 strips, thereby reducing the amount of time available for non-specific interactions to occur. Furthermore, there was a marked stimulation in test line signal in the amplifications containing template DNA (FIG. 6, bottom panel).

### Example 16: Exo probe design

This Example describes an exemplary probe design for an Exonuclease III (Exo) cleavable probe. An exemplary probe structure is shown below. The sequence below is an example probe; the sequence is designed to be complementary to the amplified target DNA. 
5'-XAAATTTCTACTTTTGGCCAGTTCTACAATTTGTTLHATATCACATG
GATGTB-3' (SEQ ID NO:1)
 Where:
X = 5' hexyl
H = THF residue
B = C3 spacer (a block for 3'-5' nuclease digestion)
L = branched modifier comprising DNP TEG and Biotin Hexyl; a phosphorothioate (PS) link between DNP TEG and Biotin hexyl moieties may be employed to ensure the stability of the inter-hapten linkage.

Probes are designed with a 3' block B (e.g. C3 spacer, e.g., propanol) prevents unwanted nuclease digestion of the probe. A branched modifier, L, is incorporated to the 5'-side of an abasic, THF residue H. In the above example, L is immediately 5' of H, but it is possible that L may be extended further from H in the S'-direction. THF residues are located with approximately 30nt of complementary sequence upstream and approximately 15nt of complementary sequence downstream.

L represents a modified cytosine (C) nucleobase, and as such should replace a C residue in the probe sequence. L is incorporated during solid-phase oligo synthesis using a commercially available phosphoramidite with the following structure (LGC LINK (Teddington, UK) item # 2150):

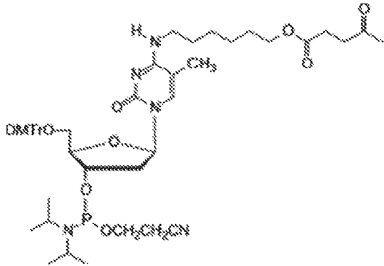

This phosphoramidite is incorporated during the course of a normal solid-phase DNA oligo synthesis; the 5'-OH of the oligo is deprotected (the DMTr protecting group is removed) and then capped with a 5' block X (e.g. C6, e.g., hexyl) before the levulinyl protecting group appended to the exocyclic amine of the modified cytosine depicted above is removed - this liberates a hydroxyl group that permits further, branching extension off the cytosine nucleobase with other phosphoramidites. Hapten-incorporating amidites, such as DNP TEG (below top, LGC LINK item # 2549) then Biotin ('Bio') hexyl (below bottom, LGC LINK item # 2109), yield a probe that is dual-labelled with haptens at the branched modifier L.

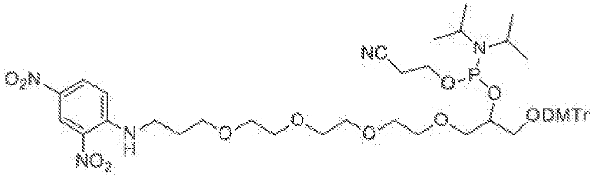

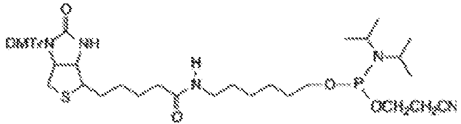

In an RPA reaction containing target amplicon, Exo III cleaves the abasic residue H of the probe, and subsequent 3'-5' digestion by Exo III liberates a mononucleotide L (with 5'-phosphate and 3'-OH) that is labelled with two distinct haptens. This dual-hapten-labelled mononucleotide is free to exit RPA coacervates and interact with antibodies on visualising particles and the test line of a LF strip.

The Exo probe design described allows modular incorporation of different haptens at the branch site L, using phosphoramidites that are commercially available. Bio/DNP, Bio/FAM and FAM/DNP labelling at L are all possible; the order of attachment of the haptens to the cytosine nucleobase of L can also be freely switched. Finally, a hapten (e.g. DNP/FAM/BIO) may be employed as the 5'-cap X of the probe in place of e.g. hexyl, in case a third distinct hapten is desired to bind and filter unprocessed probe. If the DNP-TEG phosphoramidite depicted above is employed as the 5'-cap, then an additional deprotection and capping step is utilized to remove the DMTr group and block the resultant 5'-OH with e.g. hexyl).

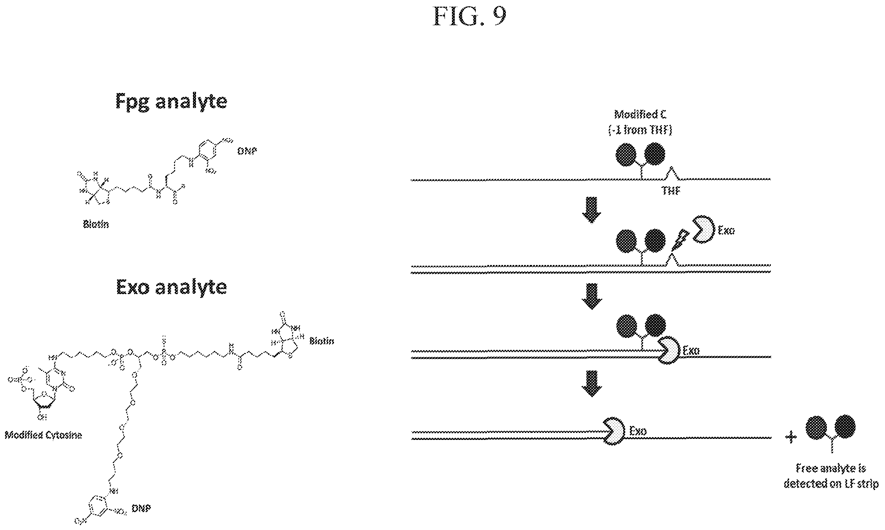

FIG. 9 shows exemplary structures of Exo probes and their use. The left panel shows a comparison of the Fpg probe analyte and Exo probe analyte - the Exo probes have a Levulnyl dC branched modifier labelled with DNP TEG and Biotin Hexyl situated one nucleotide upstream of the Tetrahydrofuran residue. The right panel shows a contemplated mechanism for how the analyte is generated in RPA. When the probe binds the complementary strand of the amplicon, Exo cuts at the THF and then nibbles back using 3' Exonuclease activity, releasing the Biotin/DNP labelled cytosine, which is then detected on strips (using anti-DNP test line and anti-biotin gold colloid or other nanoparticle)

### Reaction formulation

Briefly, Exo LF RPA reactions are incubated at 40°C for 20 minutes. RPA formulations for lateral flow (Exo) contain 420nM each of the appropriate forward and reverse primer, 120nM of the dual-hapten Exo probe, 50mM Tris Acetate pH 8.3, 100mM KOAc, 5mM DTT, 1× creatine kinase, 30µg Gp32, 30µg UvsX, 7µg UvsY, 6.5% 3kDa PEG, 5.7% trehalose, 8.6µg DNA polymerase I (S. aureus), 10µg Exonuclease III, 50mM Phosphocreatine, 2.5mM ATP, 1.8mM dNTPs and 0.5% Brij-35. Reactions are initiated by the addition of a mix containing the appropriate template and Mg(OAc)2 (22.5mM final concentration) to a final volume of 100µl.

### Continuous flow - one-step RPA lateralflow

The high sensitivity of the Exo LF probe chemistry has allowed for use in a 'continuous flow' system, where RPA nucleic acid amplification and strip detection are performed concurrently, which can reduce time to result to approximately 5 minutes from template addition (20 - 30 minutes for optimal sensitivity against low input DNA copy numbers). To make this feasible, lateral flow strips should be able to process more analyte than in the undiluted RPA LF system alone - this can be achieved by using heavyweight absorbent pad materials (e.g., Grade 320 cotton linter (Ahlstrom)) vs. the CF5 material used in endpoint detection strips. Not only does this material allow more analyte to flow on the strip, it also appears to increase the speed of flow, which reduces non-specific signals vs. other pad types. The strips also encompass a cover tape, which helps reduce delamination; common when using thick weight absorbent pads.

Aside from reducing time to result, the benefits of the continuous flow system as a whole are: 1) no need to process/dilute the amplification reaction (lowers contamination risk, simplifies potential consumable fluidics); 2) consumable fluidics are reduced (essentially the consumable is a heated chamber for RPA to occur, which contacts the lateral flow strip directly).

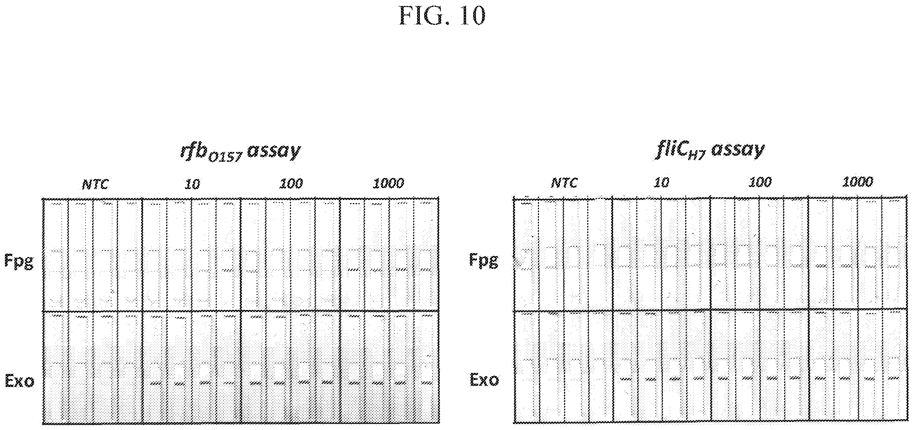

FIG. 10 shows data using the undiluted Exo LF chemistry described above in the two E. coli assays described in above examples and compared to the Fpg assay described above (numbers indicate input DNA copy number; NTC = No template control). Again, strips are added post amplification. The Exo assay exhibited higher sensitivity and a strong test line signal. Signals develop much faster than the Fpg chemistry. Additionally, while some false positive signal remains, it is much improved over the Fpg setup.

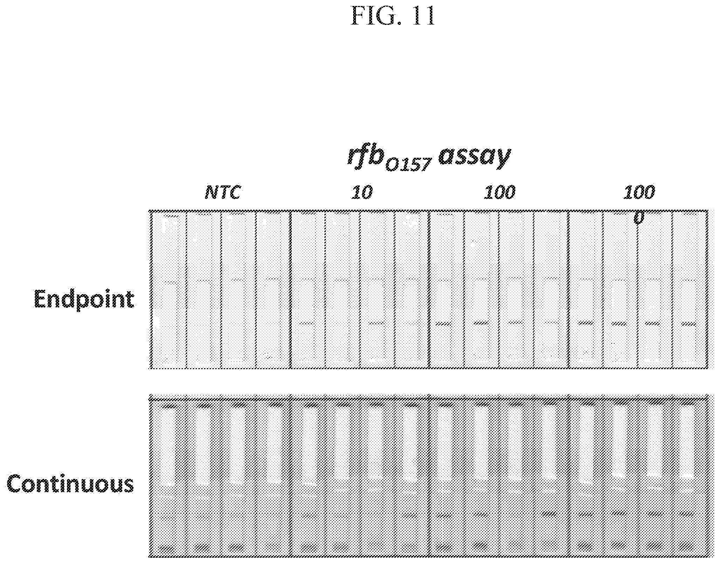

FIG. 11 shows concurrent amplification/detection using the Exo probe chemistry. Concurrent amplification and detection has the benefit that time to result is quicker (20-30 minutes) and additionally makes consumable design simpler. The data in FIG. 10 shows a comparison of endpoint detection with continuous flow. Similar sensitivity is observed between the two. False positive signals are stronger in continuous flow, which may be due to the presence of cover tape.

FIG. 12 shows an exemplary device for use in one step RPA lateral flow. The device has a reaction chamber for assay reagents and a strip chamber for detection during amplification. In the device shown in FIG. 12, there is a channel between the reaction chamber and strip chamber that allows for flow between the two chambers. In some embodiments, the chamber holds a lyophilised pellet containing RPA components. Addition of template DNA, buffer and placing the chamber onto a heat block at 40 degrees C starts amplification. In some embodiments, the chamber holds a magnetic stirrer for mixing. Amplification occurs as the reaction runs up the strip resulting in test line signal.

### OTHER EMBODIMENTS

It is to be understood that while the disclosure has been described in conjunction with the detailed description thereof, the foregoing description is intended to illustrate and not limit the scope of the disclosure, which is defined by the scope of the appended claims. Other aspects, advantages, and modifications are within the scope of the following claims.

### CLAUSES

1. A recombinase polymerase amplification composition comprising;
   a crowding agent;
   an oligonucleotide probe with a dual-hapten leaving group; and
   a nuclease enzyme.
2. The composition of clause 1, wherein the crowding agent comprises polyethylene glycol (PEG), polyvinyl alcohol (PVA), polyvinylpyrrolidone (PVP), Ficoll, or dextran.
3. The composition of clauses 1 or 2, wherein the crowding agent has a molecular weight of at least 1 kilodalton, at least 2 kilodaltons, at least 3 kilodaltons, at least 4 kilodaltons, at least 5 kilodaltons, at least 6 kilodaltons, at least 8 kilodaltons, or at least 10 kilodaltons.
4. The composition of clauses 1 to 3, wherein the crowding agent is present in the composition at a concentration of at least 15% v/v, a concentration of at least 12% v/v, a concentration of at least 10% v/v, a concentration of at least 8% v/v, at least 6% v/v, a concentration of at least 5% v/v, a concentration of at least 4% v/v, or a concentration of at least 3% v/v.
5. The composition of clauses 1 to 4, wherein the crowding agent has a viscosity profile at 20 degrees Celsius of less than or equal to 5mPa/s, less than or equal to 4mPa/s, less than or equal to 3mPa/s, less than or equal to 2mPa/s, or less than or equal to 1mPa/s.
6. The composition of clauses 1 to 5, wherein the crowding agent is PEG having a viscosity at 20 degrees Celsius is less than or equal to 3 mPa/s.
7. The composition of clauses 1 to 6, wherein the crowding agent is PEG having a molecular weight of 3 kilodaltons, and wherein the PEG is at concentration of 6.5% v/v.
8. The composition of clause 1, wherein the oligonucleotide probe comprises a dR-O-[C]n nucleotide that lacks a base linking the leaving group to the oligonucleotide.
9. The composition of clause 1, wherein the nuclease is formamidopyrimidine DNA glycosylase.
10. The composition of clauses 1 to 9, wherein the oligonucleotide probe with a dual-hapten, when cleaved by formamidopyrimidine DNA glycosylase when the oligonucleotide probe is hybridised to a complementary nucleotide sequence, releases the dual-hapten leaving group.
11. The composition of clause 1, wherein the dual-hapten leaving group comprises two immunogenic groups having different epitopes.
12. The composition of clause 11, wherein the immunogenic groups comprise a fluorescent group, an enzyme or fragment thereof, a peptide or fragment thereof, biotin.
13. The composition of clause 12, wherein the immunogenic groups are selected from the group comprising biotin, fluorescein, digoxigenin or dinitrophenyl.
14. The composition of clause 1, wherein said oligonucleotide probe is cleavable by an exonuclease, and wherein said oligonucleotide releases said dual hapten leaving group when cleaved.
15. The composition of clause 14, wherein said exonuclease is exonuclease III.
16. The composition of clause 14, wherein said oligonucleotide probe has the structure 5'X(n)ₐL(n)_{b}H(n)_{c}B3' wherein n are nucleotides a, b, and c are integers, X is a 5' hexyl, H is a THF residue, B is a C3 spacer, and L is a branched modifier comprising a plurality of haptens.
17. The composition of clause 16, wherein said haptens are selected from the group consisting of DNP, FAM, and Biotin.
18. The composition of clause 16, wherein said oligonucleotide probe comprises a phosphorothioate link between the haptens.
19. The composition of clause 16, wherein said C3 spacer is propanol.
20. A composition comprising: 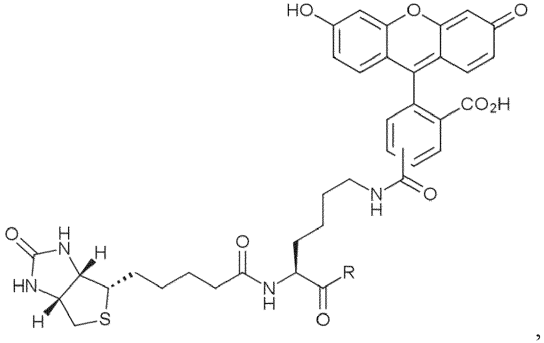 wherein R is OH or -NH(CH2)₆OH.
21. A composition comprising: 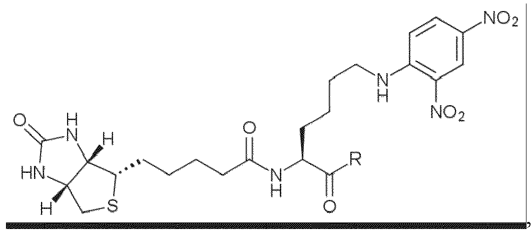 wherein R is OH or -NH(CH2)₆OH.
22. A composition comprising: 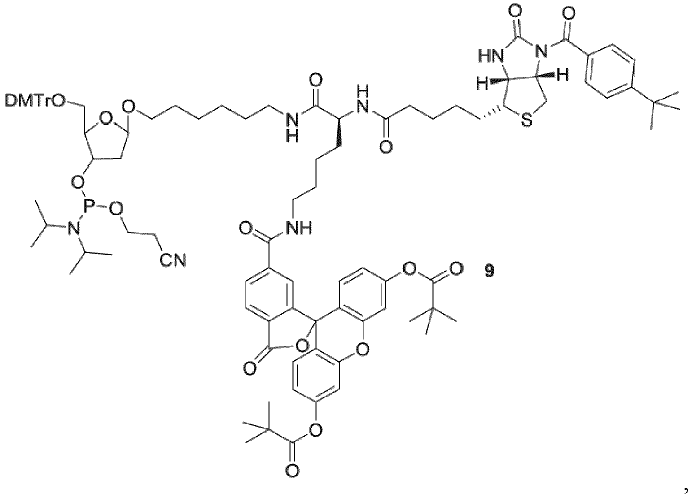 wherein where DMTr is dimethoxytrityl.
23. A composition comprising: 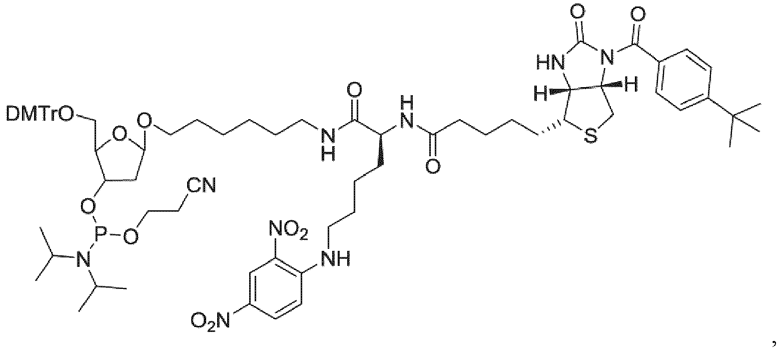 wherein DMTr is dimethoxytrityl.
24. A composition comprising: 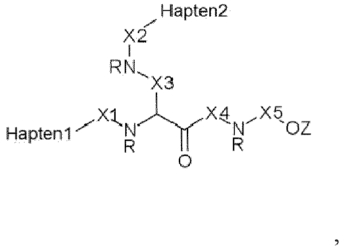 wherein:
   Hapten1 and Hapten2 are immunogenic groups according to clause 22 or 23;
   Z is selected from: (i) a C1' of an abasic ribose or deoxyribose ring within the context of an RNA or DNA oligonucleotide respectively; with a beta-configuration at the anomeric carbon atom; (ii) a phosphoramidite compound configured to link into a DNA or RNA oligonucleotide; and wherein when Z is a DNA or RNA phosphoramidite, the reactive groups of Hapten1 and Hapten2 may optionally be protected with pivaloyl; tert-butylbenzoyl; acyl; benzoyl; or isobutyryl;
   R represents hydrogen, or linear or branched C1 to C6 alkyl;
   X1, X2 and X4 are linking groups which may independently be absent, or be linear or branched C1 to C12 alkyl, which may be optionally interrupted by one or more -0-, -C(=O)- or -NR- groups;
   X3 is linear or branched C1 to C6 alkyl; and
   X5 is linear or branched C1 to C12 alkyl, which is optionally interrupted by one or more -0-, -C(=O)- or -NR- groups.
25. A device, comprising:
   a lateral flow strip, comprising
   a sample application zone;
   a reagent zone downstream of the sample application zone and in fluid communication therewith, the reagent zone comprising dried RPA reagent composition for amplifying a target nucleic acid, a binding agent specific for an amplified target nucleic acid product and a detection molecule;
   at least one test zone downstream of the reagent zone and in fluid communication therewith, the test zone comprising an immobilized capture molecule specific for the amplified target nucleic acid product; and
   a control zone downstream of the test zone.
26. The device of clause 25, wherein the dried RPA reagent composition comprises a crowding agent, a recombinase, a polymerase, a nuclease, a dual-hapten probe and a detection molecule.
27. The device of clause 26, wherein the dual-hapten probe comprises a conjugate of biotin and carboxyfluorescein (FAM) or biotin and dinitrophenyl (DNP).
28. The device of clause 25, wherein the immobilized capture molecule specific for the amplified target nucleic acid is selected from the group consisting of an anti-FAM capture molecule or an anti-DNP capture molecule.
29. The device of clause 28, wherein the anti-FAM or the anti-DNP are independently selected from the group consisting of a polyclonal antibody, a monoclonal antibody, and a functional binding fragment thereof including a FAB, a ScFv, a Fv or a DAB.
30. The device of clause 25, wherein the detection molecule is selected from the group consisting of a gold sol, a silver sol, a latex sol, a cellulose nanobead, or a carbon nanostring, and an anti-biotin capture molecule.
31. The device of clause 25, wherein the control zone comprises a binding zone that indicates correct operation of the lateral flow strip.
32. The device of clause 25, wherein the control zone comprises an anti-mouse antibody capture line.
33. A method of detecting amplification products, comprising:
   contacting a sample suspected of containing a target nucleic acid of interest with RPA reagents for amplifying the target nucleic acid, an oligonucleotide probe comprising a nucleic acid sequence complementary to the target nucleic acid and a covalently linked dual-hapten leaving group, and a nuclease;
   amplifying the target nucleic acid to produce a target nucleic acid product; and
   detecting the target nucleic acid product by detecting free dual-hapten moieties cleaved from the oligonucleotide probe hybridized to the target nucleic acid.
34. The method of clause 33, wherein the RPA reagents are located on a sample application zone of a lateral flow strip.
35. The method of clause 33, wherein nucleic acid amplification is performed on the lateral flow strip upon contact of the sample with the RPA reagents to form an nucleic acid amplification mixture.
36. The method of clause 33 wherein nucleic acid amplification is performed on the lateral flow strip without dilution or addition of other liquid to the RPA mixture.
37. The method of clause 33 wherein the dual-hapten moiety cleaved from the oligonucleotide probe is selectively captured at a test zone on the lateral flow located downstream of the sample application zone.
38. The method of clauses 33 to 37, wherein oligonucleotide probe comprising a covalently linked dual-hapten is not selectively captured at a test zone on the lateral flow strip.
39. The method of any of clauses 33 to 38, wherein the lateral flow strip comprises a test zone and a control zone, wherein the test zone comprises a binding pair member for capture of dual-hapten cleaved from oligonucleotide and the control zone comprises a binding pair member for an internal control.
40. The method of clause 39, wherein the control zone comprises an anti-mouse antibody or a fragment thereof.
41. The method of clause 39, wherein the test zone comprises an anti-DNP capture molecule or an anti-FAM capture molecule.
42. The method of clause 40 wherein the anti-FAM capture molecule is selected from the group consisting of a monoclonal antibody, a polyclonal antibody, and a functional binding fragment thereof.
43. The method of clause 42 wherein the anti-DNP capture molecule is selected from the group consisting of a monoclonal antibody, a polyclonal antibody, or a functional binding fragment thereof.
44. The method of clauses 33 to 43, wherein detecting amplification products comprises capturing dual-hapten leaving group in a test zone and labelling captured dual-hapten leaving group with a detection molecule.
45. The method of clause 44 wherein the detection molecule is selected from the group consisting of a gold sol, a silver sol, a latex sol, a cellulose nanobead, and a carbon nanostring.
46. A method comprising:
   applying a sample suspected of containing the target nucleic acid to a lateral flow strip;
   contacting the sample with a RPA reagent mixture for amplifying the target nucleic acid dried onto a reagent zone of the lateral flow strip; and
   detecting amplification products, if present, on a test zone of the lateral flow strip.
47. The method of clause 46 wherein the sample suspected of containing a target nucleic acid is applied to an application zone of a lateral flow strip.
48. The method of clause 47, wherein the RPA reagent mixture comprises a crowding agent, a recombinase, a polymerase, a nuclease, and a dual-hapten oligonucleotide probe.
49. The method of clause 48, wherein the crowding agent is selected from the group consisting of polyethylene glycol (PEG), polyvinyl alcohol (PVA), polyvinylpyrrolidone (PVP), Ficoll, and dextran.
50. The method of clause 48 or 49 wherein the crowding agent has a molecular weight of at least 1 kilodalton, at least 2 kilodaltons, at least 3 kilodaltons, at least 4 kilodaltons, at least 5 kilodaltons, at least 6 kilodaltons, at least 8 kilodaltons, or at least 10 kilodaltons.
51. The method of clause 49 or 50, or wherein the crowding agent is present in the mixture at a concentration of at least 15% v/v, at least 12% v/v final concentration, at least 10% v/v, at least 8% v/v, at least 6% v/v, at least 5% v/v, at least 4% v/v, or at least 3% v/v final concentration.
52. The method of any one of clauses 46 to 51 wherein the crowding agent has a viscosity profile at 20 degrees Celsius of less than or equal to 5mPa/s, less than or equal to 4mPa/s, less than or equal to 3mPa/s, less than or equal to 2mPa/s, or less than or equal to 1mPa/s.
53. The method of clause 46, wherein the crowding agent is PEG and has a viscosity at 20 degrees Celsius of less than or equal to 3 mPa/s.
54. The method of clause 46, wherein the crowding agent is PEG comprisng molecular weight of 3 kilodaltons and wherein the PEG is at a final concentration of 6.5% v/v.
55. The method of clause 48, wherein detecting amplification products, if present, comprises detecting a dual-hapten moiety cleaved from the oligonucleotide probe hybridized to the amplification products.
56. The method of clause 46, wherein dilution of the RPA mixture is not required prior to detection of amplification products.

## Claims

1. A device, comprising:
a lateral flow strip, comprising
a sample application zone;
a reagent zone downstream of the sample application zone and in fluid communication therewith, the reagent zone comprising a dried RPA reagent composition for amplifying a target nucleic acid, a binding agent specific for an amplified target nucleic acid product and a detection molecule;
at least one test zone downstream of the reagent zone and in fluid communication therewith, the test zone comprising an immobilized capture molecule specific for the amplified target nucleic acid product; and
a control zone downstream of the test zone.

2. The device of claim 1, wherein the dried RPA reagent composition comprises a crowding agent, a recombinase, a polymerase, a nuclease, a dual-hapten probe and a detection molecule.

3. The device of claim 2, wherein the dual-hapten probe comprises a conjugate of biotin and carboxyfluorescein (FAM) or biotin and dinitrophenyl (DNP).

4. The device of claim 1, wherein the immobilized capture molecule specific for the amplified target nucleic acid is selected from the group consisting of an anti-FAM capture molecule or an anti-DNP capture molecule.

5. The device of claim 4, wherein the anti-FAM or the anti-DNP are independently selected from the group consisting of a polyclonal antibody, a monoclonal antibody, and a functional binding fragment thereof including a FAB, a ScFv, a Fv or a DAB.

6. The device of claim 1, wherein the detection molecule is selected from the group consisting of a gold sol, a silver sol, a latex sol, a cellulose nanobead, or a carbon nanostring, and an anti-biotin capture molecule.

7. The device of claim 1, wherein the control zone comprises a binding zone that indicates correct operation of the lateral flow strip.

8. The device of claim 1, wherein the control zone comprises an anti-mouse antibody capture line.
